# EUROPEAN PATENT SPECIFICATION

(11) **EP 2 515 923 B1**
(45) Date of publication and mention of the grant of the patent: **06.11.2013**
(21) Application number: 10805539.3
(22) Date of filing: 20.12.2010
(51) Int. Cl.: A61K 36/53, A61P 25/28

(54) **ORTHOSIPHON STAMINEUS EXTRACTS WITH BENEFICIAL USE AS COGNITION ENHANCER**
EXTRAKTE AUS ORTHOSIPHON STAMINEUS ZUR VERWENDUNG ALS KOGNITIONSVERSTÄRKER
EXTRAITS D'ORTHOSIPHON STAMINEUS AYANT UNE UTILISATION BÉNÉFIQUE EN TANT QU'AGENT D'AMÉLIORATION DE LA COGNITION

(30) Priority: 21.12.2009 US 288698 P
(43) Date of publication of application: 31.10.2012
(73) Proprietor: Biotropics Malaysia Berhad, 50672 Kuala Lumpur (MY)
(72) Inventor: GEHLING, Matthias, 42799 Leichlingen (DE); GROTHE, Torsten, 44809 Bochum (DE); HANS, Joachim, 44137 Dortmund (DE); WABNITZ, Philipp, 40237 Düsseldorf (DE); V.K. GEORGE, Annie, George, 47100 Puchong Selangor (MY); TENGKU ADNAN, Tengku, Shahrir, 56000 Kuala Lumpur (MY); ZAMRI, Rozita, 70200 Seremban Negeri Sembilan (MY)
(74) Representative: Dennemeyer & Associates S.A.
(86) International application number: PCT/MY2010/000327
(87) International publication number: WO 2011/078652

(56) References cited:
- WO-A1-98/52587
- WO-A1-2008/065377

## Description

The present invention relates to the use of compounds of isopimarane diterpene type, e.g. obtainable as or from extracts from *Orthosiphon* species, especially certain enriched or purified specific compounds therefrom, as well as said compounds or extracts for use or methods of using said compounds or extracts in the management of cognitive performance; in mammals, especially humans, respectively, as well as related aspects mentioned herein.

### Compounds of isopimarane diterpene type

### Background of the invention

An issue for modem health maintenance is that due to the increasing life span and the requirements to live and be able to find orientation in the modem world, reliable and high level cognitive requirements are to be met by individuals, so that here supporting means are highly welcome. Also school and other education has a tendency to demand more and more cognitive capabilities for reaching a desired level of skills, knowledge and know-how. Thus all generations can profit from healthy and supporting foods and pharmaceuticals that promote the cognitive abilities and performance.

As a consequence, there exists a need for effective and toxicologically safe products that support and promote cognitive performance.

### Summary of the Invention

It has now been found surprisingly that compounds of the isopimarane diterpene type, obtainable e.g. as or from extracts from plants of the family Lamiaceae, such as plants of the genus *Orthosiphon*, are able to support the management of cognitive performance.

*Ortosiphon stamineus* is a herbaceous perennial plant from the family of Lamiaceae that is widely distributed throughout the tropical regions, especially in South East Asia. It can grow to 20 - 60 cm in size, with 3-16 cm narrow-oval to rhombic leaves. Its long flowers are white to bluish, with long whisker-like stamina, hence its common name Cat's whiskers. The leaves of *O*. *stamineus* are traditionally used in South East Asia as diuretic, in relation with kidney or bladder disorders. Also the use against urinary stones and as a remedy for arteriosclerosis capillary and circulatory disorders is known. Furthermore, the plant is used to treat gout, diabetes and rheumatism. The leaves contain up to 0.5 % of essential oil (mainly sesquiterpenes), together with saponins, diterpenes and flavones as main compound classes. The main active ingredients that contribute to the diuretic effect are: triterpenoid saponin, inositol, potassium salts. Other important components include Sinensetin, tetramethylscutellarein, salvigeni, orthosiphol A, B, C and D, β-sitosterol, rosmarinic acid, ursolic acid. Diterpenes present include orthosiphonone A, orthosiphonone B, neoorthosiphol A, neoorthosiphol B, and siphonols A.

The leaves are also monographed as tea drug against bladder and kidney ailments in the European Pharmacopoia (PhEur).

### Detailed Description of the Invention

The present invention, in a first embodiment, relates to a compound or mixture of compounds of the isopimarane diterpene type, an extract from a plant of the genus *Orthosiphon*, especially from the leaves thereof, comprising one or more such compounds; or mixtures of enriched compounds or single compounds obtained from such an extract in free form, in pharmaceutically or nutraceutically acceptable salt form and/or in solvate form, for use in the management of cognitive performance of a mammal, including a human.

In particular any one or more of the following compounds, or extracts comprising them, or solvates thereof, are useful according to the invention:

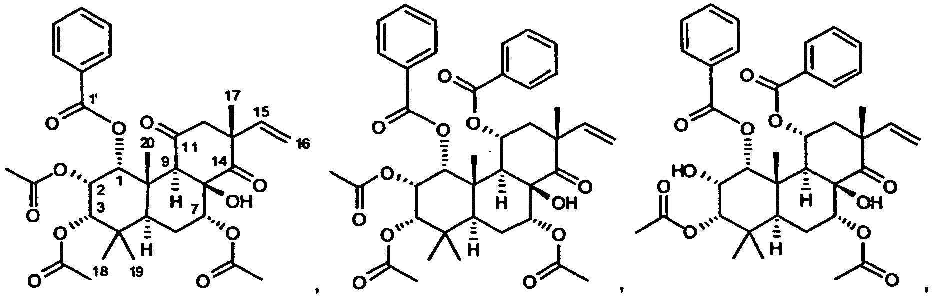

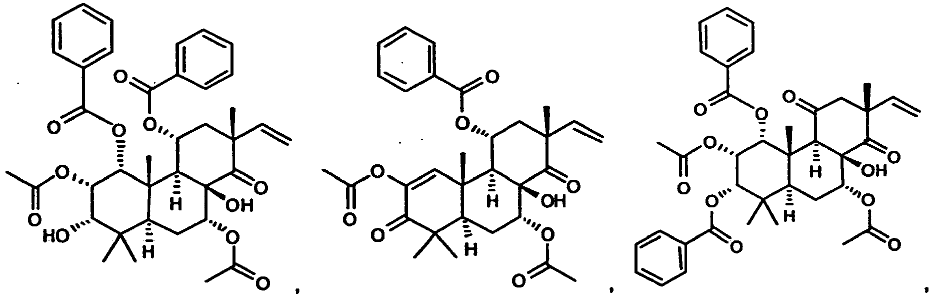

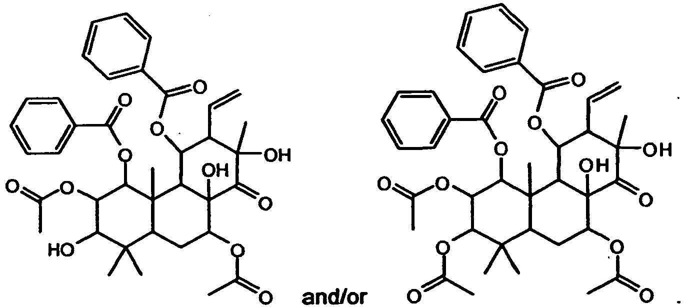

In a second embodiment, the present invention relates to a pharmaceutical composition or a nutraceutical composition comprising a compound of the isopimarane diterpene type, a mixture such of compounds or an extract comprising one or more isopimarane diterpene compounds, for use in the management of cognitive performance of a mammal including a human, together with at least one pharmaceutically or nutraceutically acceptable carrier material.

In a third embodiment, the present invention relates to a method of (prophylactically and/or therapeutically) treating a mammal, especially a human, in need of such treatment, to manage cognitive performance, comprising administering to said mammal a pharmaceutically or nutraceutically effective amount of a compound of the isopimarane diterpene type, a mixture of such compounds, and/or an extract comprising such compounds, where the compound(s) can be present in free form, in the form of a pharmaceutically acceptable salt and/or as a solvate.

In a fourth embodiment, the invention relates to a pharmaceutical formulation as in the second embodiment above, comprising one or more additional active agents.

In a fifth embodiment, the invention relates to the use of a compound, a mixture of compounds or an extract according to the first embodiment mentioned above, in the management of a cognitive disorder, or the use thereof in the manufacture of a pharmaceutical and/or nutraceutical formulation for use in said management.

Other embodiments can be deduced in the following description or from the claims.

### Figures:

Fig. 1: HPLC-UV-ELSD analysis of two extracts from *Orthosiphon Stamineus* (OS), namely OS 1 (1) [Fig. 1a)] and OS 1 (2) [Fig 1b)] as described in example 1.
Fig. 2: HPLC-UV-ELSD analysis of a food compatible extract from *Orthosiphon Stamineus* (OS) with ethanol-water 70:30 as described in Example 3.
Fig. 3: Comparison of phase separation phases obtained from a first extract obtained from *Orthosiphone Stamineus* with ethanol-water regarding yield using n-heptane (Fig. 3 a)), ethyl acetate (Fig. 3 b))and water (Fig. 3c)) phases examined by HPLC-UV-ELSD analysis, see Example 4. The numbers in square brackets [...] refer to those of the compounds mentioned in Table 4.

Among the compounds useful according to the invention, e.g. forming part of an extract from a plant of the genus *Orthosiphon*, especially from the leaves thereof, or mixtures of (e.g. enriched) compounds or single compounds (e.g. obtained from such extracts with or without chemical modification) it is preferred that any one or more of the following compounds of the formula I are present, especially up to 8 or 7 or 6 or 5 or 4 or 3 or 2 or 1 of these compounds: 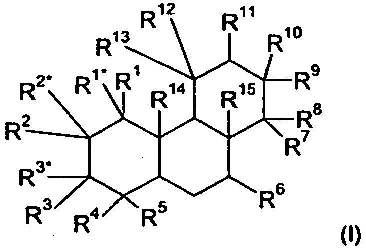 (or in another embodiment of the formula IA: 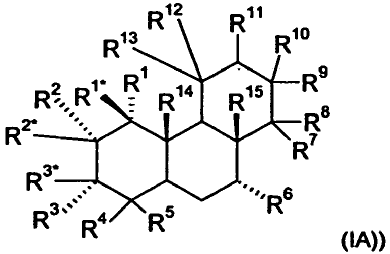 wherein, respectively,
each of R¹, R², R³, R⁶ and R¹² is, independently of the others, hydrogen, hydroxy, acyloxy, unsubstituted or substituted alkyloxy, alkenyloxy or alkynyloxy, especially C₁-C₇-alkanecarbonyl (C₂-C₈-alkanoyl) or unsubstituted or substituted C₆-C₁₂aroyl,
R^{1*} is hydrogen,
R^{2*} is hydrogen,
or R² and R^{2*} together form oxo;
or R^{1*} and R^{2*} together form a double bond,
R^{3*} is hydrogen or, together with R^{3*}, forms oxo (=O),
each of R⁴, R⁵, R¹⁰ and R¹⁴ is alkyloxy, unsubstituted or substituted alkyl, alkenyl or alkynyl, or R₄ may also be carboxyl or alkoxycarbonyl,
R⁷ and R⁸ each are hydrogen, or both together form oxo (=O),
R⁹ is hydrogen, hydroxyl, alkyl, alkenyl or alkynyl,
R" is hydrogen, hydroxyl, alkyl, alkenyl or alkynyl,
R¹³ is hydrogen or alkyl,
or R¹² and R¹³ together form oxo, and
R¹⁵ is hydroxy, acyloxy, unsubstituted or substituted alkyloxy, alkenyloxy or alkynyloxy;
or R¹¹ and R¹² together form oxo;
in free form and/or as a nutraceutically and/or pharmaceutically acceptable salt; as such and/or as solvate, respectively.

Especially preferred is a compound of the formula I, e.g. of the formula IA,
wherein:
R¹, R², R³, R⁶ are independently of each other selected from the group consisting of hydroxy, C₂-C₈alkanoyloxy and benzoyloxy;
R^{1*}, R^{2*}, R^{3*} are each hydrogen;
R⁴ is C₁-C₇alkyl;
R⁵ is C₁-C₇alkyl;
R⁷ and R⁸ together form oxo (=O);
R⁹ hydroxy, C₁-C₇alkyl or C₂-C₇alkenyl or alkynyl;
R¹⁰ is C₁-C₇alkyl;
R¹¹ is hydrogen or C₂-C₇alkenyl;
R¹² is hydroxy, C₂-C₈alkanoyloxy or benzoyloxy;
R¹³ hydrogen,
or R¹² and R¹³ together form oxo (=O);
R¹⁴ is C₁-C₇alkyl; and
R¹⁵ is hydroxyl; and/or a solvate thereof.

The term "compounds" includes the compounds in free or in salt form, or mixtures of the free or salt forms, as well as solvates (such as hydrates).

However, compounds of isopimarane diterpene type, e.g. single compounds or mixtures of compounds of the formula I, are an obligatory component of the compounds/compound mixtures or extracts useful according to the invention, that is, at least one of them must be present.

The compounds can be present in the form of one or more stereoisomers, e.g. conformation or configuration isomers.

Thus, asymmetric carbon atoms can be present in the (R)-, (S)- or (R,S)-configuration, e.g. in the (R)- or (S)-configuration. Substituents at a double bond or a ring, such as moieties bound at the central tricyclic ring in formula I or formula IA, can be present in cis- (= Z-) or trans (= E-) form. Thus the compounds useful according to the invention can be present as isomeric mixtures, e.g. racemates or mixtures of diastereomers, or as pure isomers, e.g. enantiomers or diastereomers, in general as stereoisomers.

The compound(s) and/or extracts useful according to the invention have valuable pharmacological properties. Especially, they are useful as nutraceutical and/or pharmaceutical therapeutic (including prophylactic) means for the management of cognitive performance.

These valuable properties can be shown e.g. based on the activity of the extracts or compound(s) of the formula I, with regard to management of cognitive performance, as antagonists against Adenosine receptor as the target.

Adenosine is released from metabolically active cells by facilitated diffusion and is generated extracellularly by degradation of released ATP. Ledent et al. (Ledent, C.; Vaugeois, J.-M.; Schiffmann, S. N.; Pedrazzini, T.; El Yacoubi, M. E.; Vanderhaeghen, J.-J.; Costentin, J.; Heath, J. K.; Vassart, G.; Parmentier, M.; Aggressiveness, hypoalgesia and high blood pressure in mice lacking the adenosine A2a receptor. Nature 388: 674-678, 1997) noted that it is a potent biologic mediator that modulates the activity of numerous cell types, including various neuronal populations, platelets, neutrophils and mast cells, and smooth muscle cells in bronchi and vasculature. Most of these effects help to protect cells and tissues during stress situations such as ischemia. Adenosine mediates its effects through 4 receptor subtypes: the A1 (ADORA1), A2a (ADORA2A), A2b (ADORA2B), and A3 (ADORA3) receptors. The *Adora2A* gene encodes a protein which is one of several receptor subtypes for adenosine. The activity of the encoded protein, a member of the G-protein coupled receptor family, is mediated by G proteins which activate adenylyl cyclase. ADORA2A is abundant in basal ganglia, vasculature and platelets and it is a major target of caffeine.

Huang et al. (Huang, Z.-L.; Qu, W.-M.; Eguchi, N.; Chen, J.-F.; Schwarzschild, M. A.; Fredholm, B. B.; Urade, Y.; Hayaishi, O. Adenosine A2A, but not A1, receptors mediate the arousal effect of caffeine. Nature Neurosci. 8: 858-859, 2005) found that caffeine increased wakefulness in both wildtype and Adora1-null mice, but not in Adora2a-null mice. The findings indicated that caffeine-induced wakefulness depends on adenosine A2a receptors. Evidence is given that caffeine, as a competitive inhibitor of adenosine receptors, can have nootropic effects, inducing certain changes in memory and learning. It has been found that human subjects did show increased activity in brain regions located in the frontal lobe, where a part of the working memory network is located, and the anterior cingulate cortex, a part of the brain that controls attention. The caffeinated subjects also performed better on the memory tasks.

Adenosine receptors (ADORA1 and ADORA2) have over the last decade been implicated in the modulation of cognitive functions. Despite the general view that endogenous adenosine modulates cognition through the activation of adenosine A1 receptors, evidence is now emerging on a possible role of A2A receptors in learning and memory. Present data suggest that caffeine (a nonselective adenosine receptor antagonist) as well as selective adenosine A2A receptor antagonists can improve memory performance in rodents evaluated through different tasks, see Takahashi et al., Front Biosci 13, Jan. 1 2008, 2614-32.

Adenosine receptor antagonists may also protect against memory dysfunction elicited in experimental models of aging, Alzheimer's disease, Parkinson's disease and, in spontaneously hypertensive rats, a putative genetic model of attention deficit hyperactivity disorder (ADHD).

The utility with regard to management of cognitive performance especially includes nootropic treatment, treatment for enhancing cognition outcomes, the treatment of impairment of cognitive functioning or cognitive or mental dysfunction, learning and/or memory disorders, stress-related forgetfulness, age-related mild cognitive impairment, cerebral degenerative disorders, such as such as Parkinson's Disease, Alzheimer's Disease, Huntington's disease or prionic neurodegenerative disorders such as Creutzfeld-Jacob disease and kuru disease, ischemia of the central nervous system, anxiety or depressive illness, including depression, perimenopausal depression, menopausal syndrome, post-partum depression, premenstrual syndrome, manic depression, anxiety, dementia, obsessive compulsive behaviour, ADHD (attention deficit and hyperactivity disorders), sleep disorders, irritability, impulsivity management or treatment, anger management or treatment, the improvement of learning and memory in general or treatment of withdrawal symptoms caused by termination of the use of addictive substances, like tobacco, nicotine, opioids, benzodiazepines and alcohol; or two or more such uses, as either a single agent or in combination with other agents. In some embodiments, the mammal can be a healthy mammal, e.g. human. In some embodiments, the mammal can be an aged mammal, e.g. human. The uses also include enhancing the general subjective condition, e.g. to strive for a good subjective feeling or self perception.

The extracts and/or compounds are therefore useful for pharmaceutical and/or nutraceutical purposes.

The general expressions, within the present disclosure, preferably have the following or above-mentioned meanings, where in each embodiment on, more than one or all more general expressions may, independently of each other, be replaced with the more specific definitions, thus forming (e.g. preferred) particular embodiments of the invention, respectively.

Where "a compound", "a compound of the formula I", "compounds of the formula I" or "a compound useful according to the invention" or the like is mentioned, this is intended to include a single compound, a mixture of two or more compounds of the formula I, and/or an extract comprising one or more compounds of the formula I, where the compounds of the formula I may be present in free form, in the form of a pharmaceutically and/or nutraceutically acceptable salt, in the form of a tautomer (e.g. keto/enol or the like), including a tautomeric mixture, and/or in the form of a solvate; and/or of a stereoisomer. In addition, where a compound of the formula I is mentioned, in alternative invention embodiments a compound of the formula IA is meant.

Where the prefix "lower" is used, this refers to a moiety with up to 7, e.g. 1 or (especially where at least two carbon atoms are necessary, e.g. in alkenyl or alkynyl) 2 to 7, in another embodiment of the invention 1 to 4, carbon atoms in the corresponding radical.

Lower alkyl can thus be C₁-C₇alkyl, or in an alternative embodiment of the invention C₁-C₄-alkyl, such as methyl, ethyl, n-propyl, isopropyl, n-butyl, isobutyl, sec.-butyl or tert-butyl; lower alkenyl can be C₂-C₇alkenyl, or in an alternative embodiment lower alkenyl-C₂-C₄-alkenyl, such as allyl or propenyl, and lower alkynyl can be C₂-C₇-alkynyl, or in an alternative embodiment lower alkenyl-C₂-C₄-alkenyl, such as ethynyl or propynyl. Where more than one carbon atom is present, the moieties can be linear or branched one or more times, also e.g. in alkanecarbonyl.

Lower alkanecarbonyl is e.g. C₁-C₇-alkane-carbonyl (C₁-C₇-alkyl-C(=O)-), such as acetyl, propionyl, butyroyl or the like. It is also called C₂-C₈-alkanoyl occasionally.

Acyl, e.g. in acyloxy, is preferably aroyl or lower alkanecarbonyl.

Aroyl is preferably Aryl-C(=O)- wherein Aryl is a mono- or bicyclic ring and has 6 to 14 carbon atoms, and it may be unsubstituted or substituted by one or more, e.g. 1 to 2, substituents independently selected from those mentioned below for substituted alkyl. An example is benzoyl.

Unsubstituted or substituted alkyl, e.g. in unsubstituted or substituted alkyloxy, can, in a preferred embodiment of the invention, have 1 to 20, e.g. 1 to 12 carbon atoms, or in another embodiment relates to lower alkyl. It can be unsubstituted or substituted by one or more, e.g. 1 or 2, substituents, which may be independently selected from the group comprising, e.g. consisting of, lower alkyl, e.g. methyl; halo-lower alkyl, e.g. trifuloromethyl; lower alkanoyl, e.g. acetyl; lower alkenyl; lower alkynyl; hydroxy; lower alkoxy, e.g. methoxy or ethoxy; lower-alkoxy-lower alkoxy, e.g. 2-methyloxy- or 2-ethyloxy-ethoxy; phenyl- or naphthyl-lower alkoxy; lower alkylcarbonyloxy, e.g. acetyloxy; aroyloxy, e.g. benzoyloxy; halo; amino; N-mono- or N,N-di-(lower alkyl, lower alkanecarbonyl, benzoyl or naphthoyl, with the proviso that in the case of lower alkanecarbonyl, benzoyl or naphthoyl preferably only one of these carbonyl-bound moieties is given, the other may be hydrogen or lower alkyl)-substituted amino; carboxyl (-COOH); lower alkoxycarbonyl, such as methoxycarbonyl or tert-butoxycarbonyl); phenyl-lower alkylcarbonyl; carbamoyl; N-mono- or N,N-di-lower alkylcarbamoyl; sulfamoyl; N-mono- or N,N-di-lower alkylsulfamoyl; amidino; guanidino; lower-alkanesulfonyl, e.g. methanesulfonyl; nitro and cyano. Alkyl can be linear or, if sufficient carbon atoms are present, branched.

Halo is, for example, fluoro, chloro, bromo or iodo.

Alkenyl has, for example, 2 to 20, e.g. 2 (or 3) to 12, carbon atoms and may be linear or, where sufficient carbon atoms are present, branched. Preferred is lower alkenyl, an example is allyl.

Alkynyl has, for example, 2 to 20, e.g. 2 (or 3) to 12, carbon atoms and may be linear or, where sufficient carbon atoms are present, branched. Preferred is lower alkynyl, an example is propynyl.

In alkenyloxy or alkynyloxy, alkenyl and alkynyl are defined as above.

Where the compounds of the present invention cannot be obtained directly from natural sources (e.g. by extraction), they may be obtained by chemical modification, e.g. using standard hydrolysis, hydrogenolysis, acylation, condensation, addition, deletion, substitution, reduction, oxidation, hydroxylation, carboxylation, ring-forming or comparable reactions, such as sigmatropic re-arrangements, that are known in the art. Also *in vitro* enzymatic reactions may be used.

For example, derivatives may be formed according to methods disclosed in WO 99/37600, which, in a preferred embodiment, is included herein by reference, especially with regard to the general and specific description of formation of chemically modified derivatives falling under the formula I of the present invention.

Where salt-forming groups (e.g. acidic groups or basic groups or both) are present within them, a compound of the formula I may be in the free form or in the form of a nutraceutically and/or pharmaceutically accepatable salt. The term "salt(s)", as employed herein, denotes basic salts formed with inorganic and/or organic bases, acid addition salts with inorganic and/or organic acids, and/or inner salts. Pharmaceutically and/or nutraceutically acceptable (i.e., in general non-toxic, physiologically acceptable) salts are preferred, although other salts are also useful, e.g., in isolation or purification steps which may be employed during preparation. Salts of a compound of the formula I may be formed, for example, by reacting a compound of the formula I with an amount of base or acid, such as an equivalent amount, in a medium such as one in which the salt precipitates or in an aqueous medium followed by lyophilization. Also ion exchangers can be used to form salts from free forms or free forms from salts of a compound of the formula I.

For example, compounds of the formula I which contain an acidic moiety (e.g. -COOH or-SO₃H) may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, N-methyl-D-glucamines, N-methyl-D-glucamides, t-butyl amines, and salts with amino acids such as arginine, lysine and the like. Also salts with salt-forming pharmaceutical and/or nutraceutical carrier materials are possible and encompassed by the invention.

For example, compounds of the formula I which contain a basic moiety (e.g. free, primary, secondary or tertiary amino) may form salts with a variety of organic and inorganic acids. Exemplary salts include alkanoic or alkenoic acid salts, such as acetate salts, maleic acid salts or citrate salts, sulfonic acid salts, such as methanesulfonate or toluenesulfonate salts, or inorganic salts, such as phosphates, sulfates or hydrogen halide salts, such as chlorides. Also salts with salt-forming pharmaceutical and/or nutraceutical carrier materials are possible and encompassed by the invention.

Inner salts may be formed where one or more basic and one or more addic groups are present simultaneously in a compound of the formula I.

Also salts formed between acidic groups on a molecule of a compound of the formula I and basis groups on another molecule of a compound of the formula I can be formed.

Further, a compound of the formula I (in free form, as salt or as mixture of free form and salt) may be in the form of a solvate, such as a hydrate.

Where ratios of components are given in %, this means weight %, if not indicated otherwise.

By the term "extract", either a direct extract (in liquid or preferably dried form), e.g. obtained as described below, or preferably a further enriched extract (obtainable e.g. by one or more further purification steps after extraction, e.g. chromatography, for example as described below) containing one or more, preferably two or more, e,g, 2, 3, 4, 5 or 6, compounds of the formula I is meant.

Extracts useful according to the invention are preferably prepared by a method including extraction of one or more compounds and/or mixture of compounds of the formula I from one or more plants of the genera mentioned above or below, especially from the family of Lamiaceae, such as especially *Orthosiphon stamineus,* e.g. *Orthisiphon stamineus* Benth. (also named *Clerodendranthus spicatus* (Thunb.) C.Y. Wu ex H.W. Li, *Clerodendranthus stamineus* (Benth.) Kudo, *Clerodendnim spicatum* Thunb.; *Ocimum grandiflorum* Bold.; *Orthosiphon spicatus* (Thunb.) Bak.; *Orthosiphon aristatus* (Blume) Miq., in Malaysia also called misaim kucing, kumis kucing (koemis ketjing); especially from the leaves, where preferably the extraction takes place in the presence of (this meaning that also water may be present, e.g. in an amount of up to 90 % by volume) or using a polar organic solvent or a mixture of polar solvents, where polar means a solvent that is miscible with water but more hydrophobic, such as an alcohol, e.g. a lower alkanol, such as methanol, ethanol, propanol or isopropanol, or a ketone, such as acetone, or a di-(lower alkyl)sulfoxide, such as dimethylsulfoxide, or a nitrile, such as acetonitrile, a water soluble diether, such as dioxane, an ionic liquid, or a mixture of two or more such solvents, with or without the presence of water, e.g. where the water content can, for example, lie in the range from 1 to 90% (v/v), e.g. from 3 to 80% (v/v), such as from 5 to 75% (v/v), respectively, the remainder being formed by the polar solvent or solvent mixture. Also supercritical fluids, such as nitrous oxide, sulphur dioxide, fluorocarbons or especially carbon dioxide, with or without one or more organic modifiers, e.g. co-solvents such as methanol or ethanol, halogenated hydrocarbons such as chloroform, which allow to adjust the solvating power, can be used for extraction. In a further embodiment of the invention, the extraction can be followed by a further step for enrichment, e.g. solvent partition (e.g. of an extract filled up with water and partitioned between a hydrophilic phase, e.g. with water and/or one or more polar organic solvents, and a hydrophobic phase, e.g. an essentially apolar solvent forming a separate phase in the presence of water, e.g. an alkane, such as pentanes or hexanes, or an only weekly polar solvent, such as an ester, e.g. ethyl acetate, an ether, e.g. ethyl ether, or a halogenated hydrocarbons, such as methylene chloride, and/or by chromatography, e.g. preparative high performance chromatography.

Extracts comprising one or more compounds of the formula I can be prepared from plants as mentioned above or below or plant parts, especially from the leaves.

Preferably, the total weight share of the compound or compounds of the formula I in an extract or mixture of compounds of the formula I or a purified compound of the formula I that is useful according to the invention in the final extract, mixture or compound (direct or further enriched) is in the range from 0,01 to 100 % by weight, more preferably from 0,02 to 95 %, most preferably 0,05 to 95 %, from 0.05 to 50 % or e.g. from 0.1 to 90 %., or especially of 10 % or more by weight, e.g. 30 % or more by weight, in particular 50 % or more by weight, especially 80 to 100 % by weight.

The compound(s) or extracts useful according to the invention may be used as such, in the form or pharmaceutical or nutraceutical formulations (the latter term including food additives, also named food supplements) or in the form of functional food.

Where a compound or one or more (a mixture of) compounds of the formula I, especially extracts comprising one or more compounds of the formula I, are mentioned and used as supplement, this means that the compound(s), extract or a pharmaceutical or nutraceutical formulation comprising it or them can be added to any other nutrient or pharmaceutical or nutraceutical, in specific embodiments of the invention other than nutrients, pharmaceuticals or nutraceuticals comprising probiotic micoorganisms to which extracts from *Orthosiphon aristatus* are added and/or other than nutrients, pharmaceuticals or nutraceuticals comprising a combination of extracts from *Justicia pectoralis* Jacq, *Chamomilla recutita L, Passiflora incarnate L., Plantago major* and *Zingiber officinale* Roscoe. Thus they can especially serve as food supplement. However, the compound(s), extract or formulations may also be administered as such or added to a food during a meal, e.g. via a spice or an appropriate dispenser, such as a salt cellar or pepper shaker.

"Nutraceuticals", "Functional Food", or "Functional Food products" (sometimes also called "Foodsceuticals", "Medicinal Food" or "Designer Food") for USE according to the present invention are defined as food products (including beverages) suitable for human consumption - the expression comprises any fresh or processed food having a health-promoting and/or disease-preventing property beyond the basic nutritional function of supplying nutrients, including food made from functional food ingredients or fortified with health-promoting additives, especially with effects in the management, e.g. prophylaxis or treatment, of a disease or disorder or condition as mentioned herein, that is, a compound of the formula I is used as an ingredient (especially additive) as health benefit agent, especially in an effective amount. In certain embodiments of the invention, the nutraceuticals do not comprise probiotic microorganisms and/or they do not comprise a combination of extracts from *Justicia pectoralis* Jacq, *Chamomilla recutita L, Passiflora incarnate L., Plantago major, Zingiber officinale* Roscoe together with an *Orthosiphon grandiflorus* extract. Preferably, the nutraceuticals or food additives have effects comparable to pharmaceuticals and are thus therapeutically and prophylactically active as therapeutics.

"Comprising" or "including" or "having" wherever used herein is meant not to be limiting to any elements stated subsequently to such term but rather to encompass one or more further elements not specifically mentioned with or without functional importance, that is, the listed steps, elements or options need not be exhaustive. In contrast, "containing" (an alternative word which can be used instead of "comprising" or "including" or "having" to define alternative embodiments of the invention) is used where the elements are limited to those specifically after "containing".

Where "about" is used or a specific numerical value is given without explicitly mentioning "about", this preferably means that a given value may deviate to a certain extent from the value given, e.g. preferably by ± 20 % of the given numerical value, more preferably by ± 10 %. Where numerical ranges are given, also where it is not mentioned "about" is present before any numbers marking the beginning and the end of such ranges, respectively.

"Management" of cognitive performance refers to treatment and includes both therapeutic and prophylactic treatment.

For any of the uses, the use is such that the isopimarane diterpene compound(s) (especially of formula I) or the extract comprising such compound(s) are the active ingredient, that is, they are already alone capable of achieving the intended effect.

Where "useful according to the invention" is mentioned or in any other embodiment of the invention, this especially refers to one or more of the following embodiments of the invention which can e.g. be inserted wherever "useful" is mentioned:
(1) A compound of the formula I, or a mixture of compounds of the formula I, or especially a (preferably further enriched) extract comprising one or more compounds of the formula I, for use in therapeutic (including prophylactic) treatment of a mammal, especially a human, for the management of cognitive performance.
(2) A pharmaceutical or nutraceutical composition comprising a compound of the formula I, or a mixture of compounds of the formula I, or especially a (preferably further enriched) extract comprising one or more compounds of the formula I, as active ingredient together with a pharmaceutically acceptable diluent or carrier, especially for use in the therapeutic and/or prophylactic treatment mentioned under (1).
(2') A pharmaceutical or nutraceutical composition for the treatment as mentioned under (1) comprising a compound of the formula I, or a mixture of compounds of the formula I, or especially a (preferably further enriched) extract comprising one or more compounds of the formula I, and a pharmaceutically acceptable diluent or carrier, as active ingredient supplement to a food.
(3) A functional food comprising a compound of the formula I, or a mixture of compounds of the formula I, or especially a (preferably further enriched) extract, as active ingredient for the treatment as mentioned under (1).
(4) A method for the treatment as mentioned under (1), in a subject in need of such treatment, comprising administering a pharmaceutically or nutraceutically effective amount of a compound of the formula I, a mixture of compounds of the formula I, or a (preferably further enriched) extract comprising one or more compounds of the formula I, as active ingredient, especially to an individual in need thereof.
(5) The use of a compound of the formula I, or a mixture of compounds of the formula I, or a (preferably further enriched) extract comprising one or more compounds of the formula I, as active ingredient for the manufacture of a medicament or nutraceutical or food supplement for the treatment mentioned under (1).
(6) A method or use as defined under (4), comprising co-administration, e.g. concomitantly or in sequence, of a therapeutically effective amount of compound of the formula I, or a mixture of compounds of the formula I, or a (preferably further enriched) extract comprising one or more compounds of the formula I, as active ingredient and a different pharmaceutically active compound and/or a pharmaceutically acceptable salt thereof, said different pharmaceutically active compound and/or salt thereof being especially for use in the treatment as mentioned under (1).
(7) A combination product comprising a therapeutically effective amount of a compound of the formula I, or a mixture of compounds of the formula I, or a (preferably further enriched) extract comprising one or more compounds of the formula I, as active ingredient, and a different pharmaceutically active compound and/or a pharmaceutically acceptable salt thereof, said second pharmaceutically active compound being especially for use or of use in the treatment mentioned under (1).

The functional food products or pharmaceutical products may be manufactured according to any suitable process, preferably comprising making an extract or extraction without or with further enrichment, in an alternative embodiment of the invention purification, of one or more compounds of the formula I and admixing to a functional food product or at least one nutraceutically or pharmaceutically acceptable carrier material.

Preferably, a functional food or a pharmaceutical or nutraceutical formulation comprising a compound, more preferably a compound mixture, useful according to the present invention, can be obtained by a process comprising:
(a) extraction of one or more compounds and/or mixture of compounds of the formula I from one or more plants of the genera mentioned below, especially from the family of Lamiaceae, such as especially *Orthosiphon stamineus,* e.g. *Orthisiphon stamineus* Benth. (also named *Clerodendranthus spicatus* (Thunb.) C.Y. Wu ex H.W. Li, *Clerodendranthus stamineus* (Benth.) Kudo, *Clerodendrum spicatum* Thunb., *Ocimum grandiflorum* Bold., *Orthosiphon spicatus* (Thunb.) Bak., *Orthosiphon aristatus* (Blume) Miq., in Malaysia also called misaim kucing, kumis kucing (koemis ketjing); especially from the leaves; optionally followed by a further enrichment or purification step, in particular solvent partition and/or chromatography, and
(b) mixing the resulting one or more compounds and/or mixtures of compounds as active ingredient in the preparation of the functional food product with the other constituents thereof or in order to obtain a pharmaceutical or nutraceutical formulation with one or more carrier materials or with a solvent, e.g. water or an aqueous solvent (e.g. to give a juice or dispersion or solution).

Further processing steps may precede and/or follow, such as drying (e.g. freeze-drying, spray-drying and evaporation), granulation, agglomeration, concentrating (e.g. to syrups, formed via concentration and/or with the aid of thickeners), pasteurizing, sterilizing, freezing, dissolving, dispersing, filtering, centrifuging, confectioning, packaging and the like.

When one or more compounds and/or a compound mixture or an extract according to the invention are added to a food product or pharmaceutical or nutraceutical, this also results in a functional food product or pharmaceutical or nutraceutical formulation according to the invention.

Preferably, a functional food product according to the invention comprises 0,01 to 30, e.g. 0,02 to 20, such as preferably 0.05 to 5, weight-% of a compound or mixture of compounds of the formula I or of an (especially further enriched) extract according to the invention, the rest being food and/or nutraceutically acceptable carriers and/or customary additives.

Further additives may be included, such as vitamins, minerals, e.g. in the form of mineral salts, unsaturated fatty acids or oils or fats comprising them, other extracts, or the like.

The functional food products according to the invention may be of any food type. They may comprise one or more common food ingredients in addition to the food product, such as flavours, fragrances, sugars, fruit, minerals, vitamins, stabilisers, thickeners, dietary fibers, protein, amino acids or the like in appropriate amounts, or mixtures of two or more thereof, in accordance with the desired type of food product.

Examples of basic food products and thus of functional food products according to the invention are fruit or juice products, such as orange and grapefruit, tropical fruits, banana, apple, peach, blackberry, cranberry, plum, prune, apricot, cherry, peer, strawberry, marionberry, black currant, red currant, tomato, vegetable, e.g. carrot, or blueberry juice, soy-based beverages, or concentrates thereof, respectively; lemonades; extracts, e.g. coffee, tea, green tea; dairy type products, such as milk, dairy spreads, quark, cheese, cream cheese, custards, puddings, mousses, milk type drinks and yoghurt; frozen confectionary products, such as ice-cream, frozen yoghurt, sorbet, ice milk, frozen custard, water-ices, granitas and frozen fruit purees; baked goods, such as bread, cakes, biscuits, cookies or crackers; spreads, e.g. margarine, butter, peanut butter honey; snacks, e.g. chocolate bars, muesli bars; pasta products or other cereal products, such as muesli; ready-to-serve-dishes; frozen food; tinned food; syrups; oils, such as salad oil; sauces, such as salad dressings, mayonnaise; fillings; dips; chewing gums; sherbet; spices; cooking salts; instant drink powders, such as instant coffee, instant tee or instant cocoa powder; instant powders e.g. for pudding or other desserts; or the like.

Preferably, the (e.g. pharmaceutical or nutraceutical or additive or supplement) products useful according to the invention do not contain probiotic microorganisms.

One or more other customary additives may be present, such as flavour, fragrances or other additives, such as one or more selected from stabilizers, e.g. thickeners; colouring agents, such as edible pigments or food dyes; bulking agents, such as fruit pulp, e.g. in dried form; polyols, such as xylitol, mannitol, maltitol or the like; preservatives, such as sodium or potassium benzoate, sodium or calcium carbonate or other food grade preservatives; antioxidants, such as ascorbic acid, carotionoids, tocopherols or polyphenols; mono-, oligo-or polysaccharides, such as glucose, fructose, sucrose, soy-oligosaccharides, xylooligosaccharides, galacto-oligosacharides; other artificial or natural non- or low-caloric sweeteners, such as aspartame or acesulfame; bitterness blockers; acidifiers in the form of edible acids, such as citric acids, acetic acid, lactic acid, adipic acid; flavours, e.g. artificial or natural (e.g. botanical flavours); emulsifiers; thiols, e.g. allylic thiols; diluents, e.g. maltodextrose; wetting agents, e.g. glycerol; stabilizers; coatings; isotonic agents; absorption promoting or delaying agents; and/or the like.

The one or more compounds of the formula I or compound mixtures thereof or extracts comprising them according to the invention can also be comprised in confectioned formulations to be added to foods including beverages, e.g. in the form of powders or granules, e.g. freeze-dried or spray-dried, concentrates, solutions, dispersions or other instant form, or the like.

The pharmaceutical or nutraceutical formulation (= compositions) according to the present invention can be prepared in various forms, such as granules, tablets, pills, syrups, solutions, dispersions, suppositories, capsules, suspensions, salves, lotions, emulsions, and the like. Pharmaceutical grade or food grade organic or inorganic carriers and/or diluents suitable for enteral, especially nasal or oral, parenteral (e.g. by infusion or injection) or topical use (oral formulations being preferred in all embodiments of the invention) can be used to formulate compositions containing the therapeutically-active compounds. Diluents known in the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents. The compositions may also include one or more of the following: carrier proteins such as serum albumin; buffers; fillers such as microcrystalline cellulose, lactose, corn and other starches; binding agents; sweeteners and other flavouring or fragrancing agents; coloring agents; and polyethylene glycol. Those additives are well known in the art, and are used in a variety of formulations. In certain embodiments of the invention, the pharmaceutical or nutraceutical formulations do not comprise probiotic microorganisms and/or they do not comprise a combination of extracts from *Justicia pectoralis* Jacq, *Chamomilla recutita* L, *Passiflora incarnate* L., *Plantago major, Zingiber officinale* Roscoe together with an *Orthosiphon grandifloros* extract.

By "administered" herein is meant administration of a prophylactically and/or therapeutically effective dose of a compound of the formula I or a mixture of compounds of the formula I, or an extract comprising one or more of them, to an animal, especially a human, e.g. a patient.

By "therapeutically effective dose" herein is meant a dose that produces the effects for which it is administered, especially an ameliorative or therapeutic or prophylactic effect on the conditions to be managed according to the invention of the mammal or especially human body of an individual in need of this treatment.

A mammal or human, especially being a human "patient" or "subject" for the purposes of the present invention, includes especially humans and in a broader embodiment of the invention other mammalian animals. Thus, the compound of the formula I or a mixture of compounds of the formula I, or an extract comprising one or more of them, are applicable to both humans and mammals. In the preferred embodiment the individual to be treated is a human. The mammal or human will be treated either in prophylactic or therapeutic intention, or with both intentions e.g. sequentially.

Typically, the compound of the formula I or a mixture of compounds of the formula I, or an extract comprising one or more of them, having therapeutical activity mentioned hereinbefore may be administered with at least one physiologically (= pharmaceutically or nutraceutically) acceptable carrier to a patient, as described herein. The total concentration of therapeutically active compound of the formula I or a mixture of compounds of the formula I or extracts comprising them in the formulation, as well as in a pharmaceutical formulation as such according to the invention, may vary from about 0.001-100 wt %, e.g. from 0.1 to 50 % by weight, the rest being the carrier material(s) and/or other customary additives.

The compound of the formula I or a mixture of compounds of the formula I or extracts comprising them may be formulated or administered alone or in combination with other treatments, including drugs (active agents).

E.g., for the management of cognitive performance, one or more other helpful drugs or active agents may be administered in combination with compounds or extracts useful according to the invention.

Thus, the agents of the invention can be used for the treatment of depressive symptoms in combination with: tricyclics, MAO inhibitors, SSRI's, SNRI's, NK receptor antagonists, CRF-receptor antagonists, 5HT7 receptor-antagonists, mGlu receptor agonists/antagonist/modulators, GABA-A or GABA-A/B receptor agonist/antagonists or modulators, vasopressin receptor antagonists, electroconvulsive shock, sleep deprivation, or herbal medicine with known cognitive effects such as Ginkgo biloba, St John's wort (Hypericum perforatum), Grape seeds (Vitis vinifera), Siberian ginseng (Eleutherococcus senticosus), Rhodiola rosea, Pigeon pea (Cajanus cajan), Stevia rebaudiana, Brahmi = Thyme-leafed gratiola = Water hyssop (Bacopa monnieri), Japanese comelian cherry (Comus officinalis), Magnolia officinalis, Khat (Catha edulis), Licorice (Glycyrrhiza glabra), Chinese Senna seeds = Sicklepod seeds (Cassia obtusifolia), Guggal = Guggul = Mukul myrrh tree (Commiphora wightii) or American Ginseng (Panax quinquefolius), or combinations of two or more thereof.

The agents of the invention can also be used for the treatment of anxiety-symptoms in combination with: benzodiazepines including mitochondrial benzodiazepine-ligands, 5-HT1A receptor agonists, SSRI's, SNRI's, NK receptor-antagonists, CRF receptor-antagonists, vasopressin receptor-antagonists, mGlu receptor agonists/antagonist/modulators, GABA-A or GABA-A/B receptor agonists/antagonists or modulators.

The agents of the invention can further be used for the treatment of any forms of dementia, including Alzheimer's disease (SDAT) in combination with: acetylcholine-esterase inhibitors, such as rivastigmine and donepezil, mixed acetylcholine/butyrylcholine esterase-inhibitors and nicotinic-alpha7-receptor agonists.

Moreover the agents of the invention can be used for the treatment of psychotic symptoms, including positive and negative symptoms in schizophrenia and schizoid type syndromes in combination with: any typical or atypical antipsychotic, such as clozapine or haloperidol, and nicotinic-alpha7-receptor agonists.

Furthermore the agents of the invention can be used for the treatment of bipolar disorders in combination with: any antimanic agent (e.g. Lithium, Carbamazepine, Valproate) or any atypical or typical antipsychotic.

Other psychoactive agents, e.g. agents that help in the treatment of addictive behaviour, e.g. nicotine addiction, or the like, especially in so far as they help to support the prophylaxis or treatment according to the invention intended, may also be a combination partner.

"Combination" does not necessarily mean a fixed combination but may also mean that the isopimarane diterpene compound(s) (especially of the formula I) or the extract comprising it or them may be administered in a chronically staggered manner with the combination partner(s), e.g. in the form of a kit of parts (which also is an embodiment of the invention) with other combination partners, other than those excluded hereinbefore. Preferably, the chronically staggered administration takes place such that the combination partners mutually influence, especially intensify (e.g. by way of an additive or preferably synergistic effect) their therapeutic efficiency.

Other helpful drugs or active agents may be administered, e.g. psychoactive agents, agents that help in the treatment of addictive behaviour, e.g. nicotine addiction, or the like, especially in so far as they help to support the prophylaxis or treatment according to the invention intended.

The dosage in both nutraceutical or pharmaceutical use typically is such that the amount of the compound(s) of the formula I administered to a patient is such that it is effective in modulating the targets mentioned above, or preferably a daily dose of about 0.2 to 100 g, e.g. 0.5 to 5 g is administered to a person with a weight of 70 kg per day in one or more, e.g. 1 to 3, dosages (children or persons with differing weights receive a correspondingly modified dosage).
(A) Some preferred embodiments of the invention are described below, including the Examples.
(B) In all embodiments of the invention where they are e.g. described as useful, use of compounds of the formula IA represents a particular embodiment.
(C) Another particular embodiment of the invention where isopimarane diterpene compounds are e.g. described as useful, the compound(s) is or are preferably selected from those in Table 4 in the Examples, or a solvate thereof, especially from the group consisting of Orthosiphol J, Orthosiphol H, Orthosiphol B, Orthosiphol A, Orthosiphone A and Orthosiphol D, and/or a solvate thereof.
(D) The invention also relates to an extract as descried above or below, especially from *Orthosiphon stamineus*, comprising one or more isopimarane diterpene compounds mentioned above or below, especially of the formula I, in free form, in pharmaceutically acceptable salt form or in solvate form, said extract being obtainable by extraction from *Orthosiphon stamineus* by extraction in the presence of or using an organic polar solvent or solvent mixture and/or a supercritical fluid, especially CO₂, optionally followed by solvent partition and/or chromatography. In one embodiment, the extract is from the leaves of *Orthosiphon stamineus.*
(E) The invention also relates to a compound of the formula I, mixture of compounds of the formula I and/or extract for use as described as first embodiment or elsewhere above or below, e.g. in claims 1 to 5, wherein the compound or compounds of formula I, in free form and/or in pharmaceutically acceptable salt form, are present in an amount of 10 or more % by weight, e.g. 30 or more % by weight, such as 50 % or more by weight, especially 80 to 100 % by weight.
(F) In another embodiment, the invention also relates to a compound, mixture of compounds and/or extract for use as described as first embodiment or elsewhere above or below, e.g. in claims 1 to 5, wherein the compound or compounds are in free form and/or in pharmaceutically acceptable salt form, where the use is in nootropic treatment, treatment for enhancing cognition outcomes; the treatment of impairment of cognitive functioning or cognitive or mental dysfunction, learning and/or memory disorders, stress-related forgetfulness, age-related mild cognitive impairment, cerebral degenerative disorders, ischemia of the central nervous system, anxiety or depressive illness, dementia, obsessive compulsive behaviour, ADHD (attention deficit and hyperactivity disorders), sleep disorders, irritability, impulsivity, anger; the improvement of learning and memory in general or treatment of withdrawal symptoms caused by termination of the use of addictive substances or two or more such uses, as either a single agent or in combination with one or more other active agents.
(G) In yet another embodiment, the invention also relates to A compound, mixture of compounds and/or extract for use as described as first embodiment or elsewhere above or below, e.g. in claims 1 to 5, wherein the compound or compounds are in free form and/or in pharmaceutically acceptable salt form, where the use is to enhance the general subjective condition.
(H) The invention also relates to a compound of the formula I, a mixture of compounds of the formula I and/or an extract for use as described as first embodiment or elsewhere above or below, e.g. in claims 1 to 5, wherein the compound or compounds of the formula I are present in free form and/or in pharmaceutically acceptable salt form, where the use is as a food additive.
(I). Another embodiment of the invention relates to a pharmaceutical composition or a nutraceutical composition comprising a compound of the formula I, a mixture of compounds of the formula I or an extract as described as first embodiment or elsewhere above or below, e.g. in claims 1 to 5, for use in the management of cognitive performance of a mammal including a human, together with at least one pharmaceutically or nutraceutically acceptable carrier material.
(K) Yet another embodiment of the invention relates to a pharmaceutical composition for use according to paragraph (I), comprising a further active agent.
(L) Yet a further embodiment of the invention relates to the use of a compound, a mixture of compounds or an extract as described above or below, e.g. according to any one of claims 1 to 5, in the management of a cognitive disorder according to any one of paragraphs (F), (G) or (H) mentioned above, or the use thereof in the manufacture of a pharmaceutical and/or nutraceutical formulation for use in said management.
(M) Another embodiment of the invention relates to the use of a compound, a mixture of compounds or an extract according as described above or below, e.g. according to any one of claims 1 to 5, in the management of a cognitive disorder according to any one of paragraphs (F), (G) or (H) mentioned above, or the use thereof in the manufacture of a pharmaceutical and/or nutraceutical formulation for use in said management.

(N) Yet another embodiment of the invention relates to a method of treating a mammal, including a human, in need of such treatment, to manage cognitive performance, comprising administering to said mammal a pharmaceutically or nutraceutically effective amount of a compound of the isopimarane diterpene type, a mixture of such compounds, and/or an extract comprising such compounds, as mentioned above, e.g. of the formula I, or of formula IA, or as defined under (C) in any one of claims 1 to 5, and/or an extract according to paragraph(s) (D) or (E), where the compound(s) can be present in free form, in the form of a pharmaceutically acceptable salt and/or as a solvate.

The following Examples serve to illustrate the invention without limiting its scope.

### Example 1: Preparation of crude extracts

2000 g of *Orthosiphon stamineus* leaves (commercial product: Galke, Gittelde/Harz, Germany) (OS 1) were ground into a fine powder using a lab mill (Retsch ZM200, Haan, Germany) and were extracted afterwards at room temperature twice for 30 min with 6.500 ml each of 70 % ethanol in water (v/v) using ultrasonic treatment. The solution was separated from the remaining material. The organic solvent was removed under reduced pressure at 40° C. The remaining water phase was adjusted with water to a final volume of 2000 ml and subsequently extracted four times with 2000 ml each of ethyl acetate by liquid / liquid separation. The four ethyl acetate extracts were combined (combination called OS 1 (1), with OS standing for *Orthosiphon stamineus* as source), dried (Na₂SO₄) and the solvent evaporated under reduced pressure at 40° C. The remaining water phase (OS 1 (2)) was also evaporated under reduced pressure at 40° C and the yields of dried extract were determined as shown in table 1.

**Table 1: Extract yields**

| Plant | OS No | Phases | Amount |
|---|---|---|---|
| *Orthosiphon stamineus* | OS 1 (1) | Ethyl acetate | 144.200 mg |
| *Orthosiphon stamineus* | OS 1 (2) | Water | 111.143 mg |

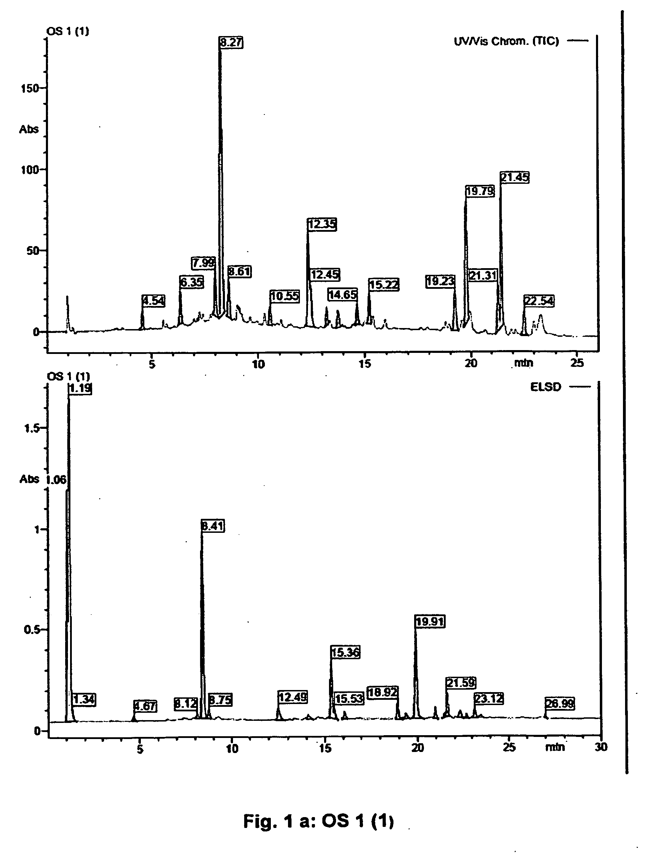

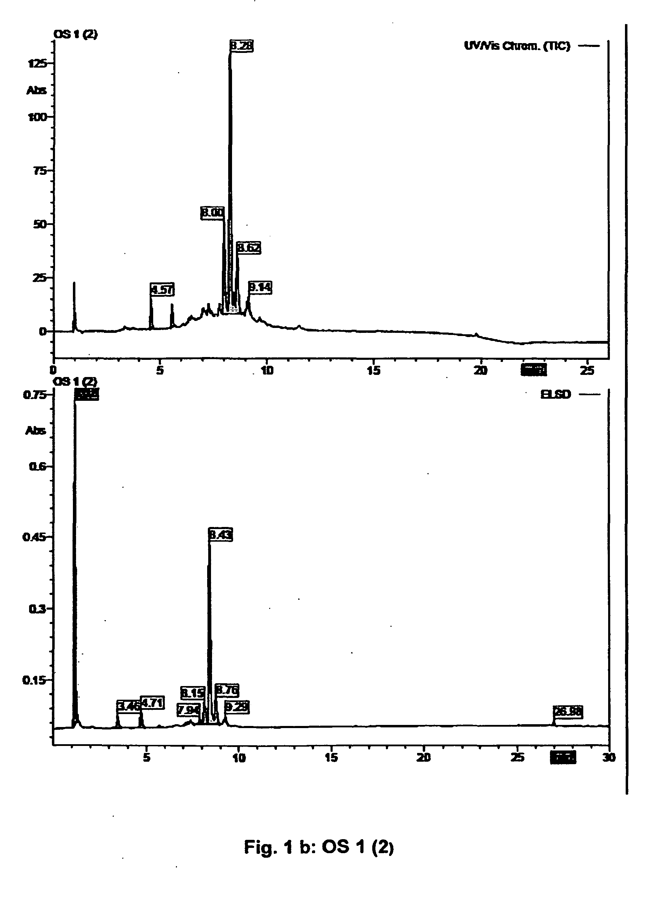

100 µg of each extract phase were analysed by HPLC-UV-ELSD (see Figure 1) by the following method.

Analytical HPLC-UV-ELSD separation was carried out on a HP 1100 Series analytical HPLC system (Agilent, Waldbronn, Germany) comprising a G 1312A binary pump system, a G 1315A diode array detector, a G 1316A column compartment, a G 1322A degasser and a G 1313A auto injector with a Nucleodur 100-5 C18ec column (column dimensions 125 x 4, Machery-Nagel, Germany), using a binary solvent system of H20 + 1.5% TFA (Solvent A) and acetonitrile +1.5% TFA (Solvent B). The gradient was set from 0% B to 100% B in 20 min at a flow of 1 ml/min at a column temperature of 40°C.

The compounds of interest are exclusively found in OS 1 (1), eluting with retention times of 12 min and above. None of the compounds of interest were detectable in OS 1 (2). Compounds found after 12 min and above in the HPLC system described above are therefore particular variants in all embodiments of the invention.

### Example 2: Preparation of pure compounds

The path information, giving the details for the overall isolation process, is given in Table 2. The initial separation step (procedure 1) was performed as MPLC separation on reversed phase material in multigram scale allowing to separate most of the matrix from the analytically detectable compounds. For all subsequent separations in preparative scale, a HPLC-setup was used comprising reversed phase separation columns with capacity for up to 200 mg material per separation. The gradients for elution were chosen according to the separation problem (table 3). Generally the systems were based on Water / Acetonitrile mixtures. On a case-wise basis, the final separation step for the isolation of pure compounds was performed on a PS1 column (Molecular filtration Chromatography, column provided by Merck, Darmstadt, Germany) using Acetonitrile as solvent under isocratic conditions. Every fraction was dried (at 40° C temperature setting) by using a vacuum concentrator and the yield was determined. For the control of every single fractionation step the resulting fractions were analysed by HPLC-UV-ELSD as described above.

**Table 2: History of isolation**

| **Procedure number** | **Starting Fraction(s)** | **Conditions of separation (if not stated otherwise)** | **Product of separation step** | **retention time period [min], yields [mg]** |
|---|---|---|---|---|
| | | **Solvent A:** H₂O + 0.1%TFA | | |
| | | **Solvent B:** Methanol + 0.1%TFA | | |
| 1 | OS 1 (1) | Chromabond P300-20 C18, 370 x 90 mm, | OS2 (9) | 80% Solvent B |
| | | Solvent A: H₂O | | |
| | | Solvent B: Methanol | | 789 mg |
| | OS 1 (1) | Chromabond P300-20 C18, 370 x 90 mm, | OS 2 (10) | Elution with 100%B |
| | | Solvent A: H₂O | | |
| | | Solvent B: Methanol | | 2960 |
| 2 | OS 2 (10) | Nucleodur 100-20 C18ec, 130 x 40 mm, Flow: 20 ml/min | OS 3 (4) | 38-41 |
| | | | | 71 |
| | OS 2 (10) | Nucleodur 100-20 C18ec, 130 x 40 mm, Flow: 20 ml/min | OS 3 (7) | 48-55 |
| | | | | 1036 |
| | OS 2 (10) | Nucleodur 100-20 C18ec, 130 x 40 mm, 20 ml/min | OS 3 (8) | 56-58 |
| | | | | 175 |
| 3 | OS 2 (9) | Nucleodur 100-20 C18ec, 130 x 40 mm, 20 ml/min | OS 4 (6) | 62-65 |
| | | | | 130 |
| 4 | | Nucleodur 100-20 C18ec, 130 x 40 mm, 20 ml/min | OS 5 (6) | 47-52 |
| | | | | 430 |
| | OS 3 (7) | Nucleodur 100-20 C18ec, 130 x 40 mm, 20 ml/min | OS 5 (7) | 53-57 |
| | | | | 95 |
| 5 | OS 3 (8) | Nucleodur 100-5 C18ec, 250 x 21 mm, 20 ml/min | OS 6 (3) | 29- 30 |
| | | | | 59 |
| 6 | OS 3 (4) + OS 4 (6) | Nucleodur 100-5 C18ec, 250 x 21 mm, 20 ml/min | OS 7 (2) | 7-8 |
| | | | | 23 |
| | OS 3 (4) + OS 4 (6) | Nucleodur 100-5 C18ec, 250 x 21 mm, 20 ml/min | OS 7 (3) | 9-10 |
| | | | | 37 |
| 12 | OS 5 (6 + 7) | Nucleosil 100-7 C8, 250 x 21 mm, 20 ml/min | OS 8 (2) | 21.5-23 |
| | | | | 136 |
| | OS 5 (6 + 7) | Nucleosil 100-7 C8, 250 x 21 mm, 20 ml/min | OS 8 (3) | 23 -24.5 |
| | | | | 187 |
| 13 | OS 5 (8) + OS 6 (3) | Nucleosil 100-7 C8, 250 x 21 mm, 20 ml/min | OS 9 (3) | 23.5 - 25 |
| | | | | 24 |
| 15 | OS 7 (2 + 3) | Nucleosil 100-7 C8, 250 x 21 mm, 20 ml/min | OS 11 (3) | 15.5 - 16.5 |
| | | | | 8 |
| | OS 7 (2 + 3) | Nucleosil 100-7 C8, 250 x 21 mm, 20 ml/min | OS 11 (4) | 17 - 18.5 |
| | | | | 18 |
| 22 | OS 11 (4 + 5) | Nucleosil 100-7 C8, 250 x 10 mm, 8 ml/min | OS 13 (4) | 17.5 - 18.5 |
| | | | | 9 |
| | OS 11 (4 + 5) | Nucleosil 100-7 C8, 250 x 10 mm, 8 ml/min | OS 13 (5) | 18.5 - 19 |
| | | | | 5 |
| 30 | OS 13 (4 + 5) | LiChrogel PS1 (10 µm), 250 x 25 mm, 4 ml/min | OS 15 (2) | 23-24.5 |
| | | | | 2 |
| | | Solvent: Acetonitrile (isocratic) | | |
| 31 | OS 9 (3) | Nucleosil 100-7 C8, 250 x 10 mm, 8 ml/min | OS 16 (3) | 13 - 14 |
| | | | | 4.5 |
| | | Solvent B: Acetonitrile + 01.% (v/v) TFA | | |
| | OS 9 (3) | Nucleosil 100-7 C8, 250 x 10 mm, 8 ml/min | OS 16 (5) | 15.5 - 18 |
| | | | | 8.8 |
| | | Solvent B: Acetonitrile + 01.% (v/v) TFA | | |
| 33 | OS 8 (2) | Nucleosil 100-7 C8, 250 x 21 mm, 20 ml/min | OS 17 (4) | 19-21 |
| | | | | 58 |
| | | Solvent B: Acetonitrile + 01.% (v/v) TFA | | |
| 34 | OS 8 (3) | Nucleosil 100-7 C8, 250 x 21 mm, 20 ml/min | OS 18 (3) | 17.5-19 |
| | | | | 23 |
| | | Solvent B: Acetonitrile + 01.% (v/v) TFA | | |
| | OS 8 (3) | Nucleosil 100-7 C8, 250 x 21 mm, 20 ml/min | OS 18 (7) | 23 - 26.5 |
| | | | | 20 |
| | | Solvent B: Acetonitrile + 01.% (v/v) TFA | | |
| 36 | OS 18 (3) | Nucleosil 100-7 C8, 250 x 10 mm, 8 ml/min | OS 19 (3) | 15 - 16 |
| | | | | 5 |
| | | Solvent B: Acetonitrile + 01.% (v/v) TFA | | |

**Table 3: solvent and gradient conditions of the procedures of table 2**

| **Procedure No.** | **Solvent System employed** | **Gradient** |
|---|---|---|
| 1 | Solvent A: H₂O | Step Gradient: |
| | Solvent B: Methanol | 20% B to 100 % B (step size 20%) |
| | | 20% B 2000 ml |
| | | 40% B 2000 ml |
| | | 60% B 1950 ml |
| | | 80% B 1950 ml |
| | | 100% B 2000 ml |
| | | 100% Isopropanol 4000 ml |
| 2 | Solvent A: H₂O + 0.1% TFA | Linear Gradient |
| | Solvent B: Methanol + 0.1% TFA | 50% B to 80% B in 60 min |
| | | 80% B to 100% B in 10 min |
| 3 | Solvent A: H₂O + 0.1% TFA | Linear Gradient |
| | Solvent B: Methanol + 0.1% TFA | 30% B to 70% B in 60 min |
| | | 70% B to 100% B in 10 min |
| 4 | Solvent A: H₂O + 0.1% TFA | Linear Gradient |
| | Solvent B: Methanol + 0.1% TFA | 60% B to 70% B in 60 min |
| | | 70% B to 100% B in 10 min |
| 5 | Solvent A: H₂O + 0.1 % TFA | Linear Gradient |
| | Solvent B: Methanol + 0.1% TFA | 50% B to 70% B in 30 min |
| | | 70% B to 100% B in 5 min |
| 6 | Solvent A: H₂O + 0.1% TFA | Linear Gradient |
| | Solvent B: Methanol + 0.1% TFA | 60% B to 65% B in 30 min |
| | | 65% B to 85% B in 5 min |
| 12; 13, 15, 22 | Solvent A: H₂O + 0.1% TFA | Linear Gradient |
| | Solvent B: Methanol + 0.1% TFA | 40% B to 70% B in 30 min |
| | | 70% B to 100% B in 5 min |
| 30 | Solvent A: Acetonitrile | Isocratic Elution |
| 31 | Solvent A: H₂O + 0.1% TFA | Linear Gradient |
| | Solvent B: Acetontrile+ 0.1% TFA | 40% B to 70% B in 30 min |
| | | 70% B to 100% B in 5 min |
| 33 | Solvent A: H₂O + 0.1% TFA | Linear Gradient |
| | Solvent B: Acetontrile+ 0.1% TFA | 40% B to 60% B in 30 min |
| | | 60% B to 100% B in 5 min |
| 34, 36 | Solvent A: H₂O + 0.1% TFA | Linear Gradient |
| | Solvent B: Acetontrile+ 0.1% TFA | 40% B to 50% B in 30 min |
| | | 50% B to 100% B in 5 min |

The separation campaign resulted in 6 pure compounds: OS 15 (2), OS 16 (3), OS 16 (5), OS 17 (4), OS 18 (7) and OS 19 (3). Details for the isolated compounds can be found in table 4.

### NMR spectroscopic data:

NMR spectra were recorded in DMSO-*d₆* on a Bruker DRX500 spectrometer at 293 K, operating at 500.13 MHz proton frequency. Structure elucidation was done by thorough interpretation of 1D and 2D NMR spectra, combined with HPLC-MS/UV data including extracted UV as well as positive and negative mode ESI spectra. The minimum NMR dataset consisted of 1D proton NMR, ¹H,¹H-gCOSY, ¹H,¹³C-gHSQC, ¹H,¹³C-gHMBC spectra.

The agreement of the experimental spectroscopic data with published data established the relative configurations of the compounds as shown in table 4. Furthermore, the relative stereochemistry of the compounds was supported by interpretation of scalar coupling constants and chemical shifts.

Analytical NMR data obtained for OS 15 (2) and OS 16 (5) were in agreement with published data by Tezuka and co-workers (T. Tezuka, P. Stampoulis, A.H. Banskota, S. Awale, K.Q. Tran, I. Saiki, S. Kadota: Constituents of the Vietnamese Medicinal Plant Orthosiphon stamineus, Chem. Pharm. Bull. 2000, 48, 1711-1719).

Analytical NMR data obtained for OS 17 (4) and OS 19 (3) were in agreement with published data by Masuda and co-workers (T. Masuda, K. Masuda, S. Shiragami, A. Jitoe, N. Nakatani: Orthosiphols A and B, novel diterpenoid inhibitors of TPA (12-O-tetradecanoylphorbol-13-acetate)-induced inflammation, from Orthosiphon stamineus. Tetrahedron 1992, 48, 6787-6792).

Analytical NMR data obtained for OS 16 (3) were in agreement with published data by Shibuya and co-workers (Y. Takeda, T. Matsumoto, H. Terao, T. Shingu, Y. Futatsuishi, T. Nohara, T. Kajimoto: Orthosiphol D and E, minor diterpenes from Orthosiphon stamineus, Phytochemistry 1993, 33, 411-415).

Analytical NMR data obtained for OS 18 (7) were in agreement with published data by Shibuya and co-workers (H. Shibuya, T. Bohgaki, T. Matsubara, M. Watarai, K. Ohashi, I. Kitagawa: Indonesian Medicinal Plants. XXII. Chemical Structures of Two New Isopimarane-Type Diterpenes, Orthosiphonones A and B, and a New Benzochromene, Orthochromene A from the Leaves of Orthosiphon aristatus (Lamiaceae), Chem. Pharm. Bull. 1999, 47, 695-698).

### Example 3: Food compatible extract

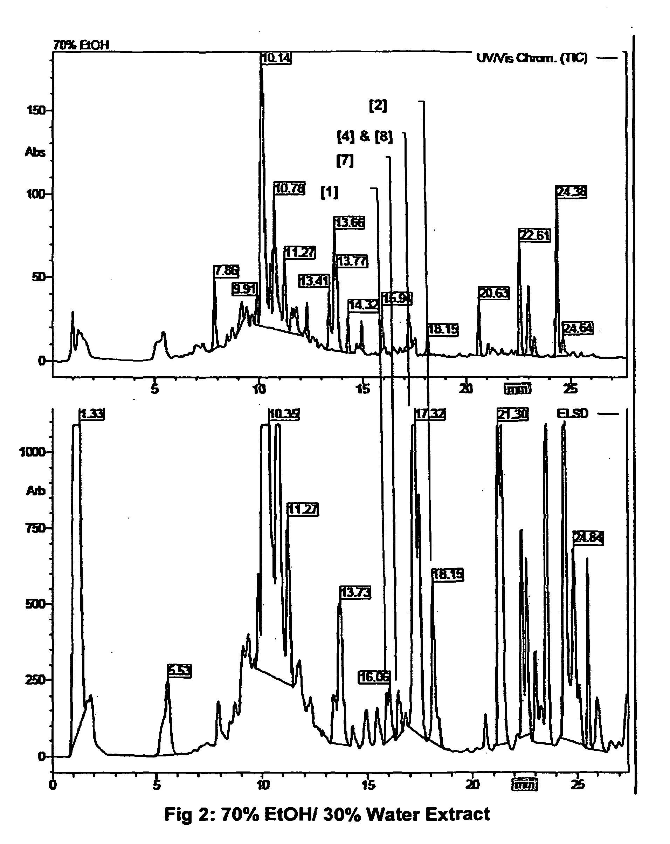

1 kg milled leaves of *O. stamineus* (same material and procedure as in example 1) were extracted twice with 2 l and 1.5 l ethanol-water 70:30. After filtration the ethanol-water extract was evaporated to dryness and the yield was determined to 128.3 g. For quality control, a 100 mg/ml sample in methanol was subjected to HPLC-UV-MS-ELSD analysis (Fig. 2)

**Table 5: quantification of compounds, calculated from the UV absorption after calibration of the HPLC with pure compounds**

| [Cmpd No.] | Peak area UV | Content [mg/kg leaves] |
|---|---|---|
| [1] | 0.302 | ~250 |
| [7] | 0.352 | ~300 |
| [4] | 3.728 | 1951.36 |
| [8] | 0.352 | 316.84 |
| [2] | 0.935 | 548.63 |

### Example 4: Dereplication

10 g milled leaves of *O*. *stamineus* (same material and procedure as in example 1) were extracted twice with 100 ml ethanol-water 90:10. After filtration the ethanol was evaporated and the remaining aqueous phase was filled up with water to a total volume of 100 ml. This aqueous sample was first degreased three times with n-heptane. Afterwards the aqueous phase was extracted three times with 100 ml ethyl acetate each. The organic phases (n-heptane and ethyl acetate) and the remaining aqueous phase were evaporated to dryness.

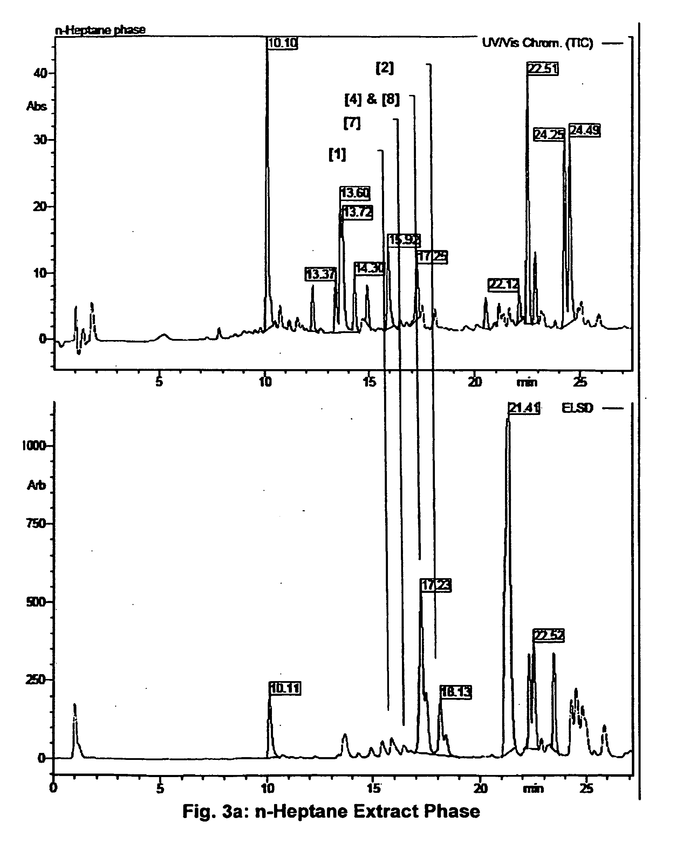

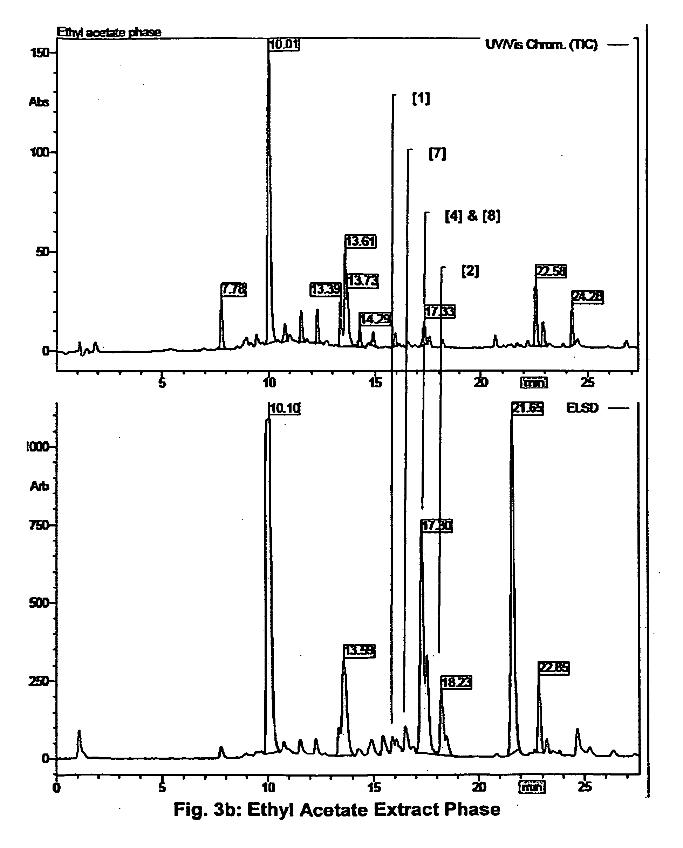

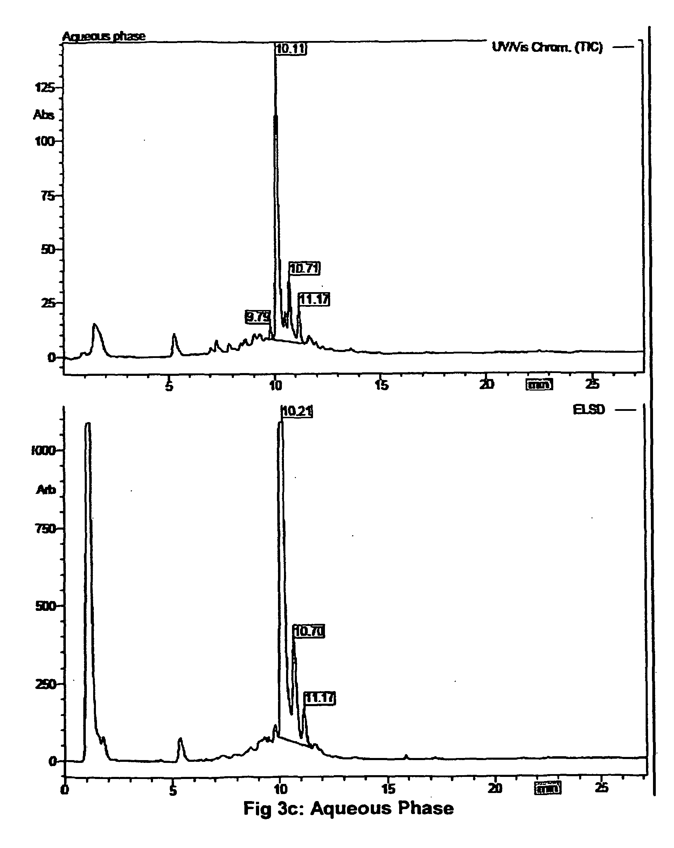

The yield of each extract phase was determined: n-heptane phase 293 mg, ethyl acetate phase 150 mg, aqueous phase 456 mg. A 10 mg/ml sample of each extract phase was subjected to HPLC-UV-MS-ELSD analysis (Fig 3). Fig 3 illustrates that the ethyl acetate phase was the most interesting one and the aqueous phase did not contain any of the desired compounds, e.g. this extract phase was free of compounds with isopimarane structure.

In order to identify structures of the compounds from the signals of the HPLC a second method was employed.

LC-MS/UV analyses for dereplication were performed using an Agilent HP1100 (Agilent, Waldbronn, Germany) liquid chromatograph coupled with a LCQ^{™} (Trademark by Finnigan) Deca XPplus mass spectrometer (Thermo Fisher Scientific, Waltham, MA, USA) in the positive and negative electrospray ionization (ESI) mode. A Waters symmetry column (Waters Symmetry® (Trademark by Waters) C18, 3.5 µm, 2.1 mm x 150 mm, Waters GmbH, Eschbom, Germany) was used as stationary phase with a flow rate of 0.4 ml/min at 40°C. Mobile phase A: 0.1 % formic acid in water, mobile phase B: 0.1 % formic acid in acetonitrile; gradient: 0-1 min. 98 % A, from 1-21 min. to 100 % B, from 21-27 min 100 % B. The UV/Vis (ultraviolet/visible light) spectra were recorded between 200-500 nm, the LC-MS (Liquid Chromatography-Mass Spectrometry coupling) spectra were recorded in the range of molecular weights between 160 and 1.600 U.

HR-ESIMS (High Resolution Electrospray Ionisation Mass Spetrometry) data were obtained on a Bruker MicroTOF (Bruker Daltonik GmbH, Leipzig, Germany) instrument, coupled with a HPLC system as described before and using sodium formiate as internal reference.

The analytical data of detected peaks were submitted to a data base alignment using a proprietary pure Natural Product reference data collection (NATPURE®, Trademark by InterMed Discovery GmbH) targeting towards the identification of compounds with emphasis on the previously isolated bioactive *Orthosiphon* metabolites. In addition, they were aligned with data reported for *O*. *stamineus* in the Chapmann & Hall Dictionary of Natural Products (Data collection version 18.2, purchased CRC Press 2008).

**Table 4: Isolated pure compounds from Orthosiphon stamineus leaves**

| **Total amount [Cmpd No]** | **Retention time [min.]** | **Structure** | **Mol. weight** | **Compound name (CAS RN)** |
|---|---|---|---|---|
| 2 mg | 14.03 (anal. method) | 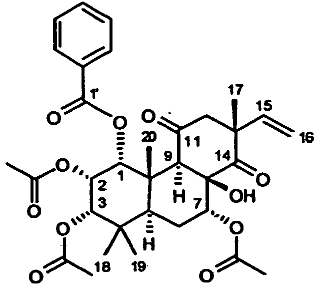 | 612.66 | Orthosiphol J |
| OS 15 (2) | | | | (316351-00-5) |
| **[1]** | 16.1 (derep. method) | | | |
| 8.8 mg | 15.91 (anal. method) | 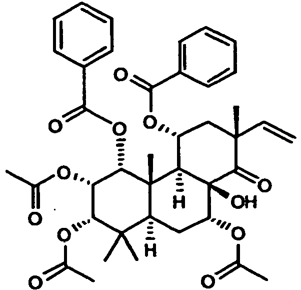 | 718.79 | Orthosiphol H |
| OS 16 (5) | | | | (238088-77-2) |
| **[2]** | 18.1 (derep. method) | | | |
| 5.1 mg | 14.85 (anal. method) | 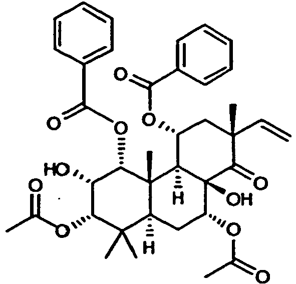 | 676.75 | Orthosiphol B |
| OS 19 (3) | | | | (144078-08-0) |
| **[3]** | | | | |
| 58.2 mg | 15.18 (anal. method) | 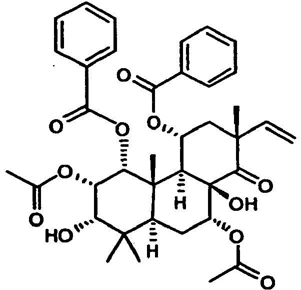 | 676.75 | Orthosiphol A |
| OS 17 (4) | | | | (142741-25-1) |
| **[4]** | 17.3 (derep. method) | | | |
| 4.5 mg | 15.21 (anal. method) | 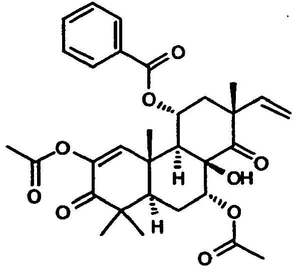 | 552.61 | Orthosiphol D |
| OS 16 (3) | | | | (149725-32-6) |
| **[5]** | | | | |
| 20.4 mg | 15.35 (anal. method) | 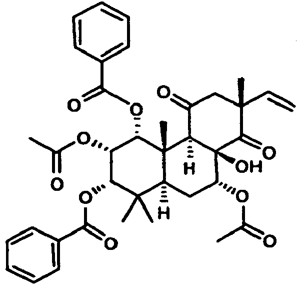 | 674.73 | Orthosipho-none A |
| OS 18 (7) | | | | |
| **[6]** | 17.5 (derep. method) | | | (237417-08-2) |
| **[7]** | 16.5 (derep. method) | 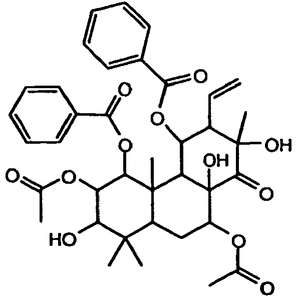 | 692.75 | Neoorthosiphol A |
| | | | | (243448-72-8) |
| **[8]** | 17.4 (derep. method) | 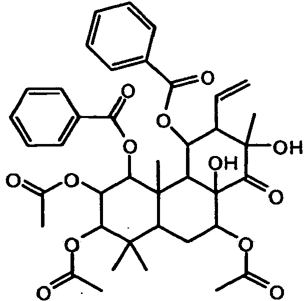 | 734.79 | Staminol A |
| | | | | (238088-74-9) |

### Example 5: Evaluation of biological activity

The biological activity may be determined with pure compounds and/or with extracts. The extracts can be prepared by any of the above outlined methods as row extracts (example 1 and/or example 3) as well as enriched or partially purified fractions according any of the procedures of example 2 and 4.

### a) ADORA2A, in vitro receptor assay

The methods employed in this study have been adapted from the scientific literature (Varani K, Gessi S, Dalpiaz A, Borea PA (1996) Br. J. Pharmacol. 117, 1693 Pharmacological and biochemical characterization of purified A2A adenosine receptors in human platelet membranes by [3H]CGS-21680 binding) to maximize reliability and reproducibility. Reference standards are run as an integral part of each assay to ensure the validity of the results obtained. Assays are performed under conditions described below. (assay number 200610, Ricerca Biosciences, LLC, Taipei, Taiwan).

| | |
|---|---|
| Source: | Human recombinant HEK-293 cells |
| Ligand: | 0.05 µM [3H] CGS-21680 |
| Vehicle: | 1% DMSO |
| Incubation Time/Temp: | 90 minutes @ 25°C |
| Incubation Buffer: | 50 mM Tris-HCl, pH 7.4, 10 mM MgCl2, 1 mM EDTA, 2 U/mL Adenosine Deaminase |
| Non-Specific Ligand: | 50 µM NECA |
| KD: | 0.064 µM |
| Bmax: | 7 pmole/mg Protein |
| Specific Binding: | 85% |
| Quantitation Method: | Radioligand Binding |

| **[Cmpd No]** | | **[%] Inhibition at 10 µM** |
|---|---|---|
| **[4]** | | 42 |
| **[5]** | | 52 |
| **[6]** | | 42 |
| **[7]** | | 64 |
| **[8]** | | 42 |
| extract according to Example 3 | 74 % (150 µg/ml) | 17 % (15 µg/ml) |

### b) Social recognition test (SRT), in vivo

The method, which detects memory enhancing activity, follows that described by Lemaire et al. (Psychopharmacology, 115, 435-440, 1994). An unfamiliar juvenile rat was introduced into the individual home cage of a mature adult rat for 5 minutes. Following this first contact (C1), the juvenile was returned to its isolation cage, until a second contact of 5 minutes with the same mature adult rat (C2), 120 minutes later was allowed.

The time the adult rat spended investigating (sniffing, grooming, licking, closely following) the juvenile at each contact was recorded. A recognition index (=C2/C1) was calculated. Under such conditions, a mature adult rat fails to recognize the juvenile as familiar, as indicated by an absence of reduction in the duration of social investigatory behaviour at C2.

12 rats were studied per group (in total 3 groups, vehicle, test compound, positive control). The test was performed blind. The test compound (e.g. the extract according example 3) was evaluated at a dose of 1g/kg administered p.o. immediately after C1 (i.e. 120 minutes before C2), and compared with a vehicle control group. The test substance was dispersed in 4% Cremophor EI, (polyoxyethylated castor oil, BASF, Ludwigshafen, Germany) in physiological saline which served as the vehicle. Donepezil (3 mg/kg) was used as reference substance with the same administration regime. Data were analyzed by comparing treated groups with appropriate control using unpaired Student's t tests.

Pre-test: For verification that the test compounds itself did not have effects on social investigation per se, a pre-test was performed with the test samples (@ 1000 mg/kg) and the vehicle control (3 groups; N=10; 120 min. before C1). As the result it can be stated that the test samples have no effect on the social investigatory behaviour itself. There is no underlying (adverse) effect that might influence the behaviour of the animals and influence the test results.

Vehicle-treated rats showed a similar level of social investigation of the familiar juvenile at the second contact, as compared with the first contact (Recognition Index = 1.06), indicating the absence of social recognition after a 2 hour delay (normal forgetting). The extract according example 3 significantly decreased the duration of investigation of the juvenile at the second contact at 1000 mg/kg, as compared with the first contact (-18%, p < 0.05). In addition, the Recognition Index was significantly decreased, as compared with vehicle controls (-23%, p < 0.05). The positive control (Donepezil, a reversible inhibitor of choline esterase which is clinically used for dementia treatment) showed an effect of -28%.

### c) Object recognition test (ORT), in vivo

The method, which detects memory enhancing activity, follows that described by Ennaceur and Delacour (Behav. Brain Res. 31, 47-59, 1988). Rats (300 - 400 g) were first habituated to the experimental enclosure, a grey plastic arena (65 x 34 x 45 cm), illuminated from above. Approximately 24 hours later, rats were individually replaced in the enclosure for 5 minutes in the presence of two identical objects (sample object) placed approximately 19 cm apart. Following this first exposure (E1), each rat was then returned to its home cage. 48 hours later, the rat was again placed in the enclosure for 3 minutes (E2) in the presence of a third copy of the sample object (familiar) and a novel object. The behavior of the rat was monitored by video.

The time spent investigating the 2 sample objects during E1 and both the novel object (E2N) and the familiar object (E2F) during E2 was recorded from videotape. A recognition index (RI = E2N-E2F/E2N+E2F) was then calculated.

Under such conditions, a rat does not show a preference for investigation of the novel object during E2, suggesting that it fails to recognize the sample object as familiar.

A similar compound application set up was used as described for the SRT in the above example. The used dose of the administered extract according to Example 3 was 100 mg/kg.

The extract according Example 3 significantly decreased the duration of investigation of the familiar object, as compared with the novel object during the second exposure (-35%, p < 0.05; RI = 0.24, p < 0.05 as compared with chance value).

## Claims

1. A compound or mixture of compounds of the isopimarane diterpene type, or an extract from a plant of the genus *Orthosiphon,* especially from the leaves thereof, comprising one or more such compounds; or mixtures of enriched compounds or single compounds obtained from such an extract, in free form, in pharmaceutically or nutraceutically acceptable salt form and/or in solvate form, for use in the management of cognitive disorders of a mammal, including a human; where the one or where appropriate more isopimarane diterpene type compounds is or are selected from the compounds of the formula I: 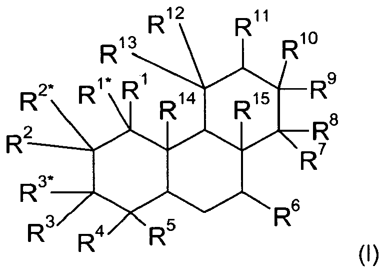 wherein
each of R¹, R², R³, R⁶ and R¹² is, independently of the others, hydrogen, hydroxy, acyloxy, unsubstituted or substituted alkyloxy, alkenyloxy or alkynyloxy, especially C₁-C₇-alkanecarbonyl (C₂-C₈-alkanoyl) or unsubstituted or substituted C₆-C₁₂aroyl,
R^{1*} is hydrogen,
R^{2*} is hydrogen,
or R²and R^{2*} together form oxo;
or R^{1*} and R^{2*} together form a double bond,
R^{3*} is hydrogen or, together with R³, forms oxo (=O),
each of R⁴, R⁵, R¹⁰ and R¹⁴ is alkyloxy, unsubstituted or substituted alkyl, alkenyl or alkynyl, or R₄ may also be carboxyl or alkyloxycarbonyl,
R⁷ and R⁸ each are hydrogen, or both together form oxo (=O),
R⁹ is hydrogen, hydroxyl, alkyl, alkenyl or alkynyl,
R¹¹ is hydrogen, hydroxyl, alkyl, alkenyl or alkynyl,
R¹³ is hydrogen or alkyl,
or R¹² and R¹³ together form oxo (=O), and
R¹⁵ is hydroxy, acyloxy, unsubstituted or substituted alkyloxy, alkenyloxy or alkynyloxy;
or R¹² and R¹³ together form oxo (=O);
in free form and/or as a nutraceutically and/or pharmaceutically acceptable salt; as such and/or as solvate, respectively.

2. A compound, compound mixture or extract for use according to claim 1, where the one or where appropriate more compounds are selected from the compounds of the formula I wherein:
R¹, R², R³, R⁶ are independently of each other hydroxy, C₂-C₈alkanoyloxy or benzoyloxy;
R^{1*}, R^{2*}, R^{3*} are hydrogen,
R⁴ C₁-C₇alkyl;
R⁵ C₁-C₇alkyl;
R⁷ and R⁸ together form oxo;
R⁹ hydroxy, C₁-C₇alkyl or C₂-C₇alkenyl or alkynyl;
R¹⁰ is C₁-C₇alkyl;
R¹¹ is hydrogen or C₂-C₇alkenyl;
R¹² is hydroxy, C₂-C₈alkanoyloxy or benzoyloxy;
R¹³ hydrogen,
or R¹² and R¹³ together form oxo;
R¹⁴ is C₁-C₇alkyl; and
R¹⁵ is hydroxyl; and/or a solvate thereof.

3. A compound, compound mixture or extract for use according to claim 1, where the compound or compounds are selected from the group consisting of compounds with the formulae 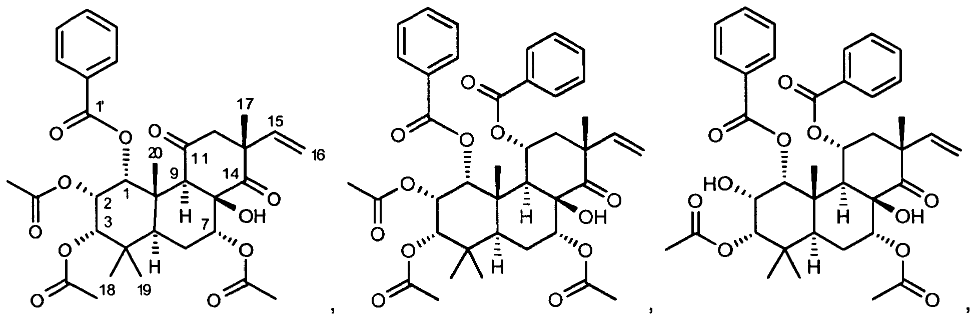 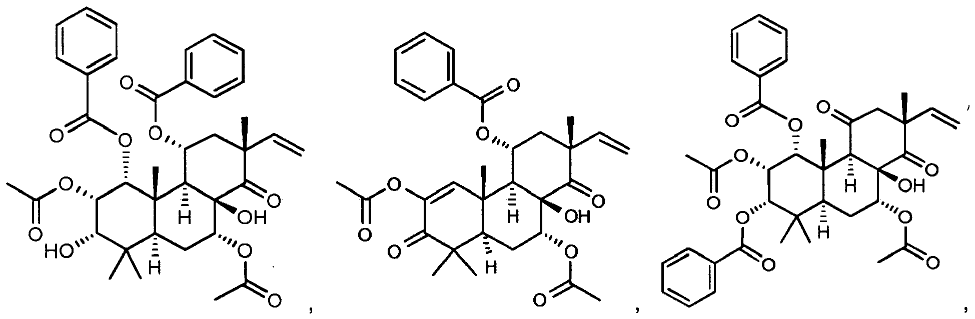 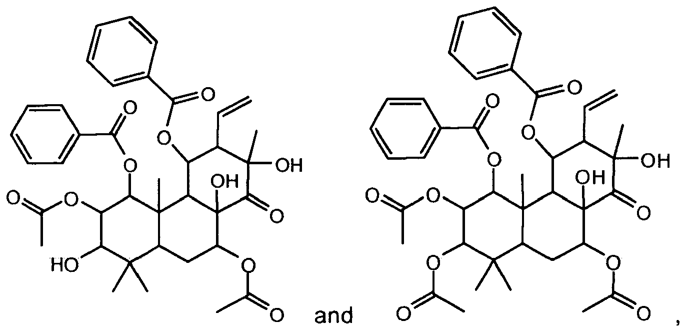 or a solvate thereof.

4. A compound, compound mixture or extract for use according to claim 1, where the compound or compounds are selected from the group consisting of Orthosiphol J, Orthosiphol H, Orthosiphol B, Orthosiphol A, Orthosiphone A and Orthosiphol D, and/or a solvate thereof.

5. An extract for use according to claim 1 from *Orthosiphon stamineus,* comprising one or more compounds mentioned in any one of claims 1 to 4, in free form, in pharmaceutically acceptable salt form or in solvate form, said extract being obtainable by extraction from Orthosiphon stamineus by extraction in the presence of or using an organic polar solvent or solvent mixture and/or a supercritical fluid, especially CO₂, optionally followed by solvent partition and/or chromatography.

6. An extract for use according to claim 5, obtainable from the leaves of *Orthosiphon stamineus.*

7. A compound of the formula I, mixture of compounds of the formula I and/or extract for use according to any one of claims 1 to 4, wherein the compound or compounds of formula I, in free form and/or in pharmaceutically acceptable salt form, are present in an amount of 10 or more % by weight.

8. A compound, mixture of compounds and/or extract for use according to any one of claims 1 to 4, wherein the compound or compounds are in free form and/or in pharmaceutically acceptable salt form, where the use is in nootropic treatment, treatment for enhancing cognition outcomes; the treatment of impairment of cognitive functioning or cognitive or mental dysfunction, learning and/or memory disorders, stress-related forgetfulness, age-related mild cognitive impairment, cerebral degenerative disorders, anxiety or depressive illness, dementia, obsessive compulsive behaviour, attention deficit and hyperactivity disorders, sleep disorders,; or the improvement of learning and memory in general or two or more such uses, as either a single agent or in combination with one or more other active agents.

9. A compound, mixture of compounds and/or extract for use according to any one of claims 1 to 4, wherein the compound or compounds are in free form and/or in pharmaceutically acceptable salt form, wherein the use is in the management of a cognitive disorder based on ischemia of the nervous system, irritability, impulsivity, anger or withdrawal symptoms caused by the termination of the use of addictive substances.

10. A compound, mixture of compounds and/or extract for use according to any one of claims 1 to 4, wherein the compound or compounds are in free form and/or in pharmaceutically acceptable salt form, where the use is via oral administration.

11. A compound of the formula I, mixture of compounds of the formula I and/or extract for use according to any one of claims 1 to 4, wherein the compound or compounds of the formula I are present in free form and/or in pharmaceutically acceptable salt form, where the use is as a food additive.

12. A pharmaceutical composition or a nutraceutical composition comprising a compound of the formula I or a mixture of compounds of the formula I in free form, in pharmaceutically acceptable salt form and/or in solvate form, or an extract according to any one of claims 1 to 4, for use in the management of cognitive disorders of a mammal including a human, together with at least one pharmaceutically or nutraceutically acceptable carrier material, with our without the presence of one or more additional active agents.

13. The use of a compound, a mixture of compounds or an extract according to any one of claims 1 to 4, in the management of a cognitive disorder according to any one of claims 1, 8, 9 or 10, or the use thereof in the manufacture of a pharmaceutical and/or nutraceutical formulation for use in said management.

## Patentansprüche

1. Verbindung oder Mischung von Verbindungen des Isopimaranditerpen-Typs oder ein Extrakt einer Pflanze der Gattung *Orthosiphon,* insbesondere aus den Blättern davon, umfassend eine oder mehrere solche Verbindungen; oder Mischungen von angereicherten Verbindungen oder einzelne Verbindungen, die aus einem solchen Extrakt erhalten wurden, in freier Form, in Form eines pharmazeutisch oder nutrazeutisch akzeptablen Salzes und/oder in Solvatform zur Verwendung beim Management kognitiver Störungen eines Säugetiers, einschließlich des Menschen; wobei die eine oder ggf. die mehreren Verbindungen vom Isopimaranditerpen-Typ aus den Verbindungen der Formel I ausgewählt ist bzw. sind: 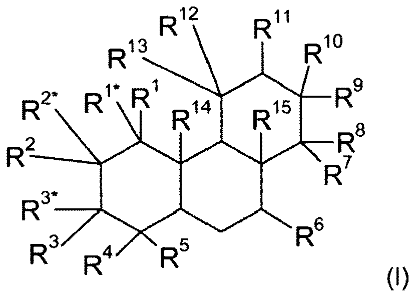 wobei
jedes von R¹, R², R³, R⁶ und R¹² unabhängig von den anderen Wasserstoff, Hydroxy, Acyloxy, unsubstituiertes oder substituiertes Alkyloxy, Alkenyloxy oder Alkynyloxy, insbesondere C₁-C₇-Alkancarbonyl(C₂-C₈-alkanoyl) oder unsubstituiertes oder substituiertes C₆-C₁₂-Aroyl, ist,
R^{1*} Wasserstoff ist,
R^{2*} Wasserstoff ist,
oder R² und R^{2*} gemeinsam Oxo bilden;
oder R^{1*} und R^{2*} gemeinsam eine Doppelbindung bilden,
R^{3*} Wasserstoff ist oder gemeinsam mit R³ Oxo(=O) bildet,
jedes von R⁴, R⁵, R¹⁰ und R¹⁴ Alkyloxy, unsubstituiertes oder substituiertes Alkyl, Alkenyl oder Alkynyl ist,
oder R⁴ ebenfalls Carboxyl oder Alkyloxycarbonyl sein kann,
R⁷ und R⁸ jeweils Wasserstoff sind oder beide gemeinsam Oxo(=O) bilden,
R⁹ Wasserstoff, Hydroxyl, Alkyl, Alkenyl oder Alkynyl ist,
R¹¹ Wasserstoff, Hydroxyl, Alkyl, Alkenyl oder Alkynyl ist,
R¹³ Wasserstoff oder Alkyl ist,
oder R¹² und R¹³ gemeinsam Oxo(=O) bilden, und
R¹⁵ Hydroxy, Acyloxy, unsubstituiertes oder substituiertes Alkyloxy, Alkenyloxy oder Alkynyloxy ist;
oder R¹² und R¹³ gemeinsam Oxo(=O) bilden;
in freier Form und/oder als nutrazeutisch und/oder pharmazeutisch akzeptables Salz; an sich und/oder als Solvat.

2. Verbindung, Mischung von Verbindungen oder Extrakt zur Verwendung nach Anspruch 1, wobei die eine oder ggf. die mehreren Verbindungen aus den Verbindungen der Formel I ausgewählt sind, wobei:
R¹, R², R³, R⁶ unabhängig voneinander Hydroxy, C₂-C₈-Alkanoyloxy oder Benzoyloxy sind;
R^{1*}, R^{2*}, R^{3*} Wasserstoff sind,
R⁴ C₁-C₇-Alkyl ist;
R⁵ C₁-C₇-Alkyl ist;
R⁷ und R⁸ gemeinsam Oxo bilden;
R⁹ Hydroxy, C₁-C₇-Alkyl oder C₂-C₇-Alkenyl oder Alkynyl ist;
R¹⁰ C₁-C₇-Alkyl ist;
R¹¹ Wasserstoff oder C₂-C₇-Alkenyl ist;
R¹² Hydroxy, C₂-C₈-Alkanoyloxy oder Benzoyloxy ist;
R¹³ Wasserstoff ist,
oder R¹² und R¹³ gemeinsam Oxo bilden;
R¹⁴ C₁-C₇-Alkyl ist; und
R¹⁵ Hydroxyl ist; und/oder ein Solvat davon.

3. Verbindung, Verbindungsmischung oder Extrakt zur Verwendung nach Anspruch 1, wobei die Verbindung oder Verbindungen ausgewählt sind aus der Gruppe bestehend aus Verbindungen mit den Formeln: 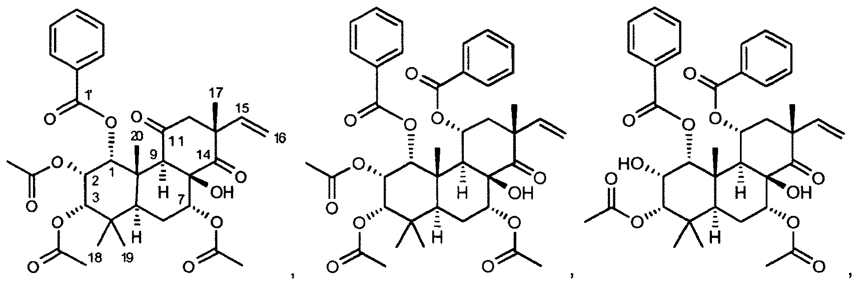 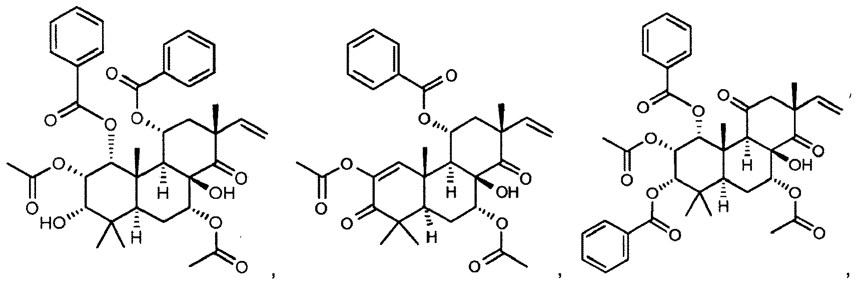 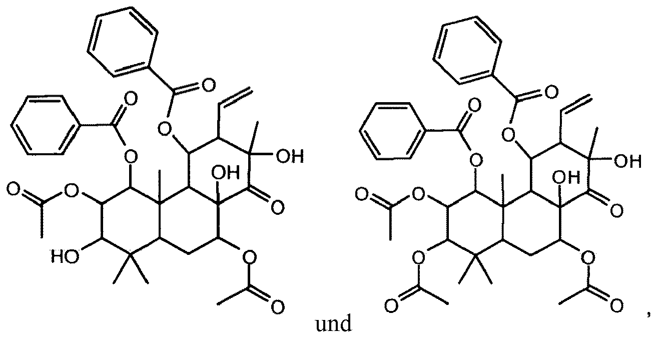 oder ein Solvat davon.

4. Verbindung, Verbindungsmischung oder Extrakt zur Verwendung nach Anspruch 1, wobei die Verbindung oder Verbindungen aus der Gruppe bestehend aus Orthosiphol J, Orthosiphol H, Orthosiphol B, Orthosiphol A, Orthosiphon A and Orthosiphol D ausgewählt sind und/oder ein Solvat davon.

5. Extrakt zur Verwendung nach Anspruch 1 aus *Orthosiphon stamineus*, umfassend eine oder mehrere Verbindungen, die in einem der Ansprüche 1 bis 4 erwähnt ist bzw. sind, in freier Form, ein Form eines pharmazeutisch akzeptablen Salzes oder in Solvatform, wobei das Extrakt durch Extraktion aus *Orthosiphon stamineus* mittels Extraktion in der Gegenwart oder unter Verwendung von einem organischen polaren Lösungsmittel oder einer Mischung von organischen polaren Lösungsmitteln und/oder einem superkritischen Fluid, insbesondere CO₂, optional gefolgt von einer Lösungsmittelabtrennung und/oder Chromatographie, erhalten wird.

6. Extrakt zur Verwendung nach Anspruch 5, erhältlich aus den Blättern von *Orthosiphon stamineus.*

7. Verbindung der Formel I, Mischung von Verbindungen der Formel I und/oder Extrakt zur Verwendung nach einem der Ansprüche 1 bis 4, wobei die Verbindung oder Verbindungen der Formel I in freier Form und/oder in Form eines pharmazeutisch akzeptablen Salzes in einer Menge von 10 Gew.-% oder mehr vorhanden ist bzw. sind.

8. Verbindung, Mischung von Verbindungen und/oder Extrakt zur Verwendung nach einem der Ansprüche 1 bis 4, wobei die Verbindung oder Verbindungen in freier Form und/oder in Form eines pharmazeutisch akzeptablen Salzes vorliegen, wobei die Verwendung in der nootropischen Behandlung, der Behandlung zur Verbesserung von kognitiven Ergebnissen; der Behandlung einer Beeinträchtigung der kognitiven Funktion oder einer kognitiven oder mentalen Dysfunktion, von Lern- und/oder Gedächtnisstörungen, stressbedingter Vergesslichkeit, altersbedingter leichter kognitiver Beeinträchtigung, zerebralen degenerativen Störungen, Angst oder depressive Erkrankung, Demenz, obsessivem Zwangsverhalten, Aufmerksamkeitsdefizit- und Hyperaktivitätsstörungen, Schlafstörungen; oder zur Verbesserung des Lernens und des Gedächtnisses im Allgemeinen ist oder zwei oder mehrere solcher Verwendungen entweder als einzelnes Mittel oder in Kombination mit einem oder mehreren anderen Wirkstoffen.

9. Verbindung, Mischung von Verbindungen und/oder Extrakt zur Verwendung nach einem der Ansprüche 1 bis 4, wobei die Verbindung oder Verbindungen in freier Form und/oder in Form eines pharmazeutisch akzeptablen Salzes vorliegen, wobei die Verwendung beim Management einer kognitiven Störung auf der Basis von Ischämie des Nervensystems, Reizbarkeit, Impulsivität, Wut oder Entzugssymptomen ist, die durch Beendigung der Verwendung von Suchtmitteln verursacht sind.

10. Verbindung, Mischung von Verbindungen und/oder Extrakt zur Verwendung nach einem der Ansprüche 1 bis 4, wobei die Verbindung oder Verbindungen in freier Form und/oder in Form eines pharmazeutisch akzeptablen Salzes vorliegen, wobei die Verwendung über orale Verabreichung erfolgt.

11. Verbindung der Formel I, Mischung von Verbindungen der Formel I und/oder Extrakt zur Verwendung nach einem der Ansprüche 1 bis 4, wobei die Verbindung oder Verbindungen der Formel I in freier Form und/oder in Form eines pharmazeutisch akzeptablen Salzes vorliegen, wobei die Verwendung als Lebensmittelzusatzstoff erfolgt.

12. Pharmazeutische Zusammensetzung oder nutrazeutische Zusammensetzung, umfassend eine Verbindung der Formel I oder eine Mischung von Verbindungen der Formel I in freier Form, in Form eines pharmazeutisch akzeptablen Salzes und/oder in Solvatform oder ein Extrakt nach einem der Ansprüche 1 bis 4 zur Verwendung beim Management kognitiver Störungen eines Säugetiers, einschließlich des Menschen, gemeinsam mit zumindest einem pharmazeutisch oder nutrazeutisch akzeptablen Trägermaterial mit oder ohne Gegenwart eines oder mehrerer weiterer Wirkstoffe.

13. Verwendung einer Verbindung, einer Mischung von Verbindungen oder eines Extrakts nach einem der Ansprüche 1 bis 4 beim Management einer kognitiven Störung nach einem der Ansprüche 1, 8, 9 oder 10 oder Verwendung davon bei der Herstellung einer pharmazeutischen und/oder nutrazeutischen Formulierung zur Verwendung beim Management.

## Revendications

1. Composé ou mélange de composés du type isopimarane-diterpène, ou un extrait d'une plante du genre *Orthosiphon,* en particulier de ses feuilles, comprenant un ou plusieurs de ces composés ; ou des mélanges de composés enrichis ou de composés isolés obtenus à partir d'un tel extrait, sous forme libre, sous forme de sel acceptable du point de vue pharmaceutique ou nutraceutique, et/ou sous forme de solvate, pour utilisation dans la gestion des troubles cognitifs chez un mammifère, y compris un humain ; dans lequel un composé ou, lorsque c'est approprié, plusieurs composés de type isopimarane-diterpène est ou sont choisis parmi les composés de formule I : 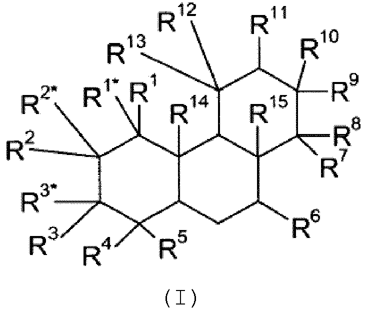 dans laquelle
chacun de R¹, R², R³, R⁶ et R¹² est, indépendamment des autres, l'hydrogène, hydroxy, acyloxy, alkyloxy substitué ou non substitué, alcényloxy ou alcynyloxy, en particulier (alcane en C₁ à C₇) carbonyl-alcanoyle en C₂ à C₈ ou aroyle en C₆ à C₁₂ substitué ou non substitué,
R^{1*} est l'hydrogène,
R^{2*} est l'hydrogène,;
ou bien R² et R^{2*} forment ensemble oxo ;
ou bien R^{1*} et R^{2*} forment ensemble une double liaison,
R^{3*} est l'hydrogène ou, conjointement avec R³, forme oxo (=O), chacun de R⁴, R⁵, R¹⁰ et R¹⁴ est alkyloxy, alkyle substitué ou non substitué, alcényle ou alcynyle, ou bien R⁴ peut aussi être carboxyle ou alkyloxycarbonyle,
chacun de R⁷ et R⁸ est l'hydrogène, ou bien ils forment ensemble oxo (=O),
R⁹ est l'hydrogène, hydroxyle, alkyle, alcényle ou alcynyle,
R¹¹ est l'hydrogène, hydroxyle, alkyle, alcényle ou alcynyle, R¹³ est l'hydrogène ou alkyle,
ou bien R¹² et R¹³ forment ensemble oxo (=O), et
R¹⁵ est hydroxy, acyloxy, alkyloxy substitué ou non substitué, alcényloxy ou alcynyloxy ;
ou bien R¹² et R¹³ forment ensemble oxo (=O) ;
sous forme libre et/ou sous forme de sel acceptable du point de vue pharmaceutique ou nutraceutique ; tel quel et/ou sous forme de solvate, respectivement.

2. Composé, mélange de composés ou extrait pour utilisation selon la revendication 1, dans lequel un composé ou, lorsque c'est approprié, plusieurs composés, sont choisis parmi les composés de formule I dans laquelle :
R¹, R², R³ et R⁶ sont indépendamment les uns des autres hydroxy,
alcanoyloxy en C₂ à C₈ ou benzoyloxy ;
R^{1*}, R^{2*} et R^{3*} sont des hydrogènes,
R⁴ est un alkyle en C₁ à C₇ ;
R⁵ est un alkyle en C₁ à C₇ ;
R⁷ et R⁸ forment ensemble oxo ;
R⁹ est hydroxy, alkyle en C₁ à C₇ ou alcényle ou alcynyle en C₂ à C₇ ;
R¹⁰ est un alkyle en C₁ à C₇ ;
R¹¹ est l'hydrogène ou un alcényle en C₂ à C₇ ;
R¹² est hydroxy, alcanoyloxy en C₂ à C₈ ou benzoyloxy ;
R¹³ est l'hydrogène,
ou bien R¹² et R¹³ forment ensemble oxo ;
R¹⁴ est un alkyle en C₁ à C₇ ; et
R¹⁵ est hydroxyle ;
et/ou un solvate de ceux-ci.

3. Composé, mélange de composés ou extrait pour utilisation selon la revendication 1, dans lequel le composé ou les composés sont choisis dans le groupe constitué par les composés de formules 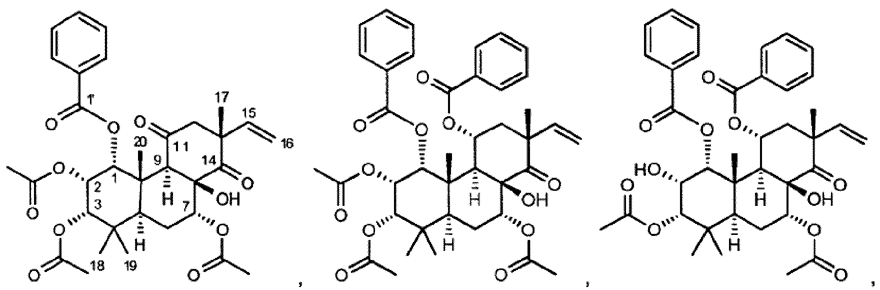 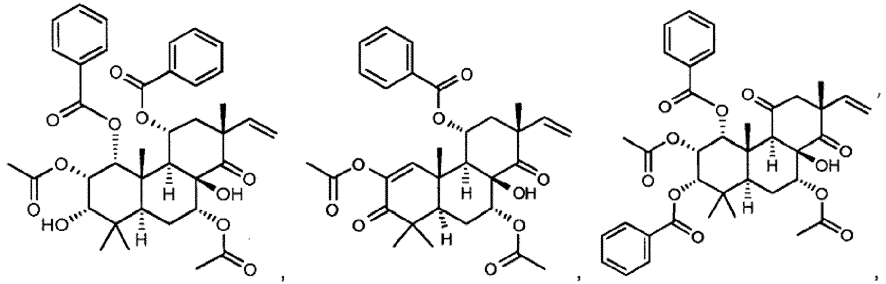 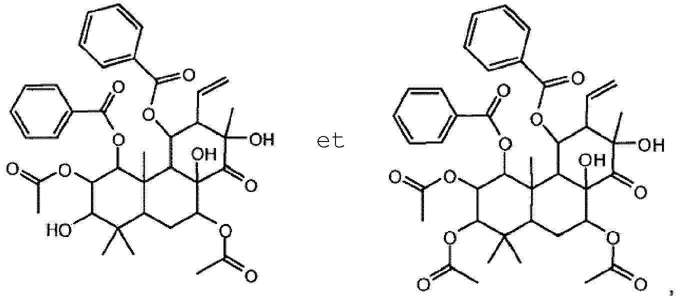 ou un solvate de ceux-ci.

4. Composé, mélange de composés ou extrait pour utilisation selon la revendication 1, dans lequel le composé ou les composés sont choisis dans le groupe constitué par Orthosiphol J, Orthosiphol H, Orthosiphol B, Orthosiphol A, Orthosiphone A et Orthosiphol D, et/ou un solvate de ceux-ci.

5. Extrait pour utilisation selon la revendication 1 obtenu à partir d'*Orthosiphon stamineus,* comprenant un ou plusieurs composés mentionnés dans l'une quelconque des revendications 1 à 4, sous forme libre, sous forme pharmaceutiquement acceptable ou sous forme de solvate, ledit extrait pouvant être obtenu par extraction à partir d'Orthosiphon stamineus par extraction en présence ou par utilisation d'un solvant ou d'un mélange de solvants organiques polaires et/ou d'un fluide supercritique, en particulier CO₂, éventuellement suivie d'une séparation des solvants et/ou d'une chromatographie.

6. Extrait pour utilisation selon la revendication 5, pouvant être obtenu à partir des feuilles d'*Orthosiphon stamineus.*

7. Composé de formule I, mélange de composés de formule I et/ou extrait pour utilisation selon l'une quelconque des revendications 1 à 4, dans lequel le composé ou les composés de formule I, sous forme libre et/ou sous forme de sel pharmaceutiquement acceptable, sont présents en une quantité de 10 % en poids ou plus.

8. Composé, mélange de composés et/ou extrait pour utilisation selon l'une quelconque des revendications 1 à 4, dans lequel le composé ou les composés sont sous forme libre et/ou sous forme de sel pharmaceutiquement acceptable, dans lequel l'utilisation réside dans un traitement nootropique, un traitement pour améliorer les évolutions cognitives ; le traitement d'une altération du fonctionnement cognitif ou d'un dysfonctionnement cognitif ou mental, de troubles de l'apprentissage et/ou de la mémoire, de l'inattention liée au stress, d'une altération cognitive légère liée à l'âge, de troubles dégénératifs cérébraux, de l'anxiété ou d'une maladie dépressive, de la démence, d'un comportement obsessionnel compulsif, d'un déficit de l'attention et de troubles d'hyperactivité, de troubles du sommeil ; ou l'amélioration de l'apprentissage et de la mémoire en général ou deux ou plus de ces utilisations, soit sous la forme d'un agent unique soit en combinaison avec un ou plusieurs autres agents actifs.

9. Composé, mélange de composés et/ou extrait pour utilisation selon l'une quelconque des revendications 1 à 4, dans lequel le composé ou les composés sont sous forme libre et/ou sous forme de sel pharmaceutiquement acceptable, dans lequel l'utilisation réside dans la gestion d'un trouble cognitif basé sur une ischémie du système nerveux, de l'irritabilité, de l'impulsivité, de la colère ou de symptômes de sevrage dus à l'arrêt de l'utilisation de substances addictives.

10. Composé, mélange de composés et/ou extrait pour utilisation selon l'une quelconque des revendications 1 à 4, dans lequel le composé ou les composés sont sous forme libre et/ou sous forme de sel pharmaceutiquement acceptable, dans lequel l'utilisation est une administration par voie orale.

11. Composé de formule I, mélange de composés de formule I et/ou extrait pour utilisation selon l'une quelconque des revendications 1 à 4, dans lequel le composé ou les composés de formule I sont sous forme libre et/ou sous forme de sel pharmaceutiquement acceptable, dans lequel l'utilisation réside dans un additif alimentaire.

12. Composition pharmaceutique ou composition nutraceutique comprenant un composé de formule I ou un mélange de composés de formule I sous forme libre, sous forme de sel pharmaceutiquement acceptable et/ou sous forme de solvate, ou un extrait selon l'une quelconque des revendications 1 à 4, pour utilisation dans la gestion de troubles cognitifs chez un mammifère, y compris un humain, conjointement avec au moins un matériau formant véhicule acceptable du point de vue pharmaceutique ou nutraceutique, en présence ou en l'absence d'un ou plusieurs agents actifs additionnels.

13. Utilisation d'un composé, d'un mélange de composés ou d'un extrait selon l'une quelconque des revendications 1 à 4, dans la gestion d'un trouble cognitif selon l'une quelconque des revendications 1, 8, 9 et 10, ou utilisation de celui-ci dans la fabrication d'une formulation pharmaceutique et/ou neutraceutique pour utilisation dans ladite gestion.
